# EUROPEAN PATENT APPLICATION

(11) **EP 2 543 372 A1**
(43) Date of publication of application: **09.01.2013**
(21) Application number: 11173379.6
(22) Date of filing: 08.07.2011
(51) Int. Cl.: A61K 31/4412, A61P 35/00

(54) **Medicament for the treatment of liver cancer**

(71) Applicant: Helmholtz-Zentrum für Infektionsforschung GmbH, 38124 Braunschweig (DE)
(72) Inventor: Zender, Lars, Prof. Dr., 38124 Braunschweig (DE); Rudalska, Ramona, Dr., 38124 Braunschweig (DE); Dauch, Daniel, 38124 Braunschweig (DE)
(74) Representative: Taruttis, Stefan Georg

(57) **Abstract**

The invention provides a pharmaceutical composition comprising Sorafenib in combination with an inhibitor of a specific kinase inhibitor as a medicament for the treatment or prevention of liver cancer.

## Description

The present invention relates to a medicament and to a pharmaceutical composition, respectively, which is suitable for the treatment or prevention of liver cancer, especially hepatocellular carcinoma (HCC) in human patients.

Further, the invention relates to a method for the treatment of liver cancer by administration of the pharmaceutical composition to a patient, and to a process for producing a medicament or pharmaceutical composition, respectively, which is suitable for the treatment of liver cancer.

### State of the art

Today, the only curative options for the treatment of liver cancer are surgical resection or liver transplantation. However, at the time of diagnosis the majority of patients present with advanced tumor growth and are therefore not eligible for these treatment options. Liver cancer is a primarily chemoresistant tumor and only recently Llovet and coworkers described the multikinase inhibitor Sorafenib as the first systemic treatment which can prolong survival of patients with HCC. However, the treatment is costly and only yields a survival advantage of less than three month. Therefore, there is an urgent clinical need for the development of medicaments which increase efficiency over Sorafenib.

### Objects of the invention

It is an object of the invention to provide a pharmaceutical composition for the treatment of liver cancer with an increased efficacy.

### General description of the invention

The invention achieves these objects by providing a pharmaceutical composition, the composition comprising Sorafenib and an inhibitor of Mapk14 (p38α kinase inhibitor) as a medicament for the treatment and/or prevention of liver cancer. The inhibitor of Mapk14 activity is a pharmaceutical active agent which in combination with Sorafenib is used as a medicament for the treatment and/or prevention of liver cancer. In the combination, Sorafenib and the inhibitor of Mapk14 can be provided for joint administration, e.g. as a composition containing both Sorafenib and an inhibitor of Mapk14, or Sorafenib and the inhibitor of Mapk14 can be provided for separate administration, e.g. firstly Sorafenib and secondly an inhibitor of Mapk14, or firstly an inhibitor of Mapk14 and secondly Sorafenib. Accordingly, the invention provides a combination of Sorafenib with an inhibitor of Mapk14 as a medicament for the treatment and/or prevention of liver cancer for joint or separate administration as well as a composition comprising a combination of Sorafenib and of an inhibitor of Mapk14, e.g. each of Sorafenib and an inhibitor of the activity of Mapk14 contained in a separate formulation for use in separate administration, or a formulation containing both Sorafenib and an inhibitor of the activity of Mapk14 in combination.

It has been demonstrated during the preparation of the invention that an agent which inhibits the activity of Mapk14 increases the efficacy of Sorafenib, as a medicament in the treatment or prevention of liver cancer. As the inhibition of the activity of Mapk14 can be obtained by the inhibition of the expression of Mapk14 gene product or protein, inhibitors of the translation of the mRNA encoding Mapk14, and inhibitors of Mapk14 protein activity, e.g. kinase inhibitors, are comprised in the group of inhibitors of the activity of Mapk14 which are used in a combination with Sorafenib for use as a medicament for the treatment or prevention of liver cancer.

Inhibitors of the activity of Mapk14, especially of the expression of Mapk14, are comprised in the group of small regulatory RNAs such as small inhibitory RNAs (siRNA), short hairpin RNA (shRNA) and microRNAs (miRNA), having a nucleic acid sequence hybridizing under physiological conditions, e.g. within a liver cell, to the mRNA encoding Mapk14 to reduce or inhibit the presence of Mapk14 in a hepatocyte or a liver cancer cell through RNA interference and accordingly inhibit the activity of Mapk14. Small regulatory RNA molecules comprise or consist of the group containing at least one of the following inhibitory RNAs: SEQ ID NO: 1 to SEQ ID NO: 1364.

ShRNA molecules, e.g. contained in microRNA molecules, hybridize to the mRNA encoding Mapk14. The Mapk14 encoding gene gives rise to four mRNAs, which are regarded as splice products. The splice product mRNAs are given as SEQ ID NO: 1365 to SEQ ID NO: 1368. The shRNA of the invention through RNA interference reduce the expression, and hence the activity of Mapk14 gene product. Alternatively, the inhibitory RNAs can be contained in classical antisense oligonucleotides for targeting the same mRNA regions for suppression of Mapk14 expression.

The specificity of shRNA, especially when the sequence encoding the shRNA was contained in a microRNA can also be shown in an vitro test using the reduction of the expression of a reporter gene product from a fusion gene, which produces a fusion mRNA that contains the coding sequence for both the reporter gene and for Mapk14. In this assay, it can be found that the mRNA of the fusion gene, which mRNA comprises the coding sequence for Mapk14 in combination with the coding sequence for the reporter gene, is reduced in the presence of an shRNA which is specific for the mRNA encoding Mapk14, whereas a control encoding an mRNA of the reporter gene only did not show a reduction of the reporter gene expression in the presence of an shRNA hybridizing to the mRNA encoding Mapk14.

The inactivation of Mapk14 by inhibiting or reducing the expression of Mapk14 in hepatocytes or liver cancer cells in experimental animals, both by suppression of the expression of Mapk14 via continuous expression of an shRNA specific for the mRNA encoding Mapk14 in the mouse model, and by administration of a pharmaceutical composition, in which the pharmaceutical active agent consists of an shRNA specific for the mRNA encoding Mapk14 of the mouse reduces the present liver cancer or prevents the generation of liver cancer upon administration of agents which in the absence of the combination of a Mapk14 inhibitor and Sorafenib would induce a liver cancer.

The reduction or repression of Mapk14 activity by reducing or suppressing the expression of Mapk14 using the continuous expression of an shRNA which is specifically directed against the mRNA encoding Mapk14, or by administration of a pharmaceutical composition containing as the active ingredient or agent an shRNA specifically directed against the mRNA encoding Mapk14 in the animal model reduces a previously established liver cancer with increased efficacy upon administration of Sorafenib in comparison to the treatment with Sorafenib as the only pharmaceutical active agent, i.e. Sorafenib without the combination with an agent reducing Mapk14 activity.

In a preferred embodiment, the inhibitor of the activity of Mapk14 is at least one of the group comprising or consisting of inhibitors which have preference or specificity for inactivating the Mapk14 gene product, which compounds are given in the following table 1.

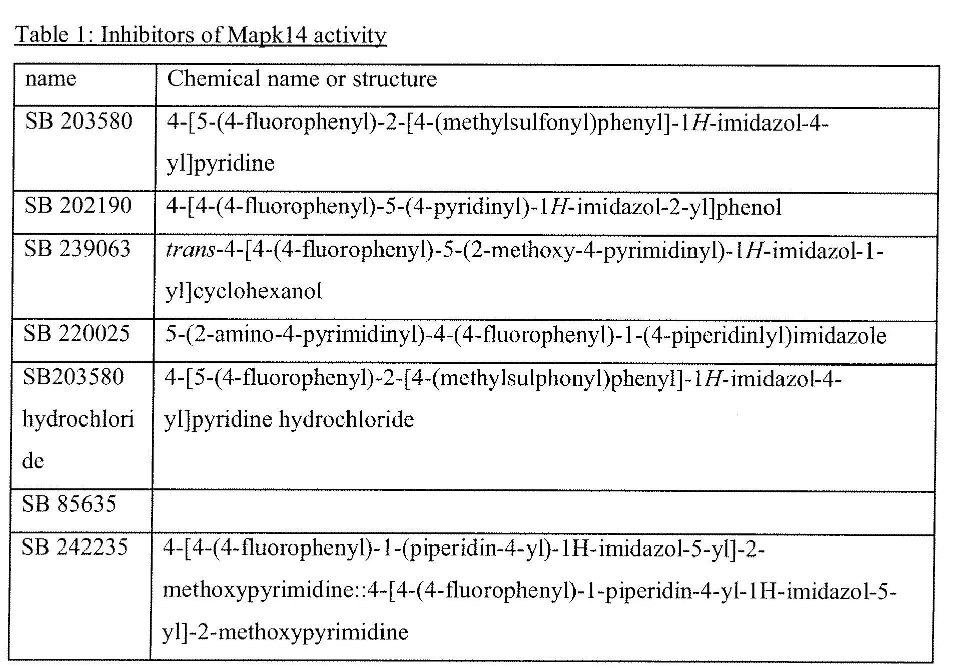

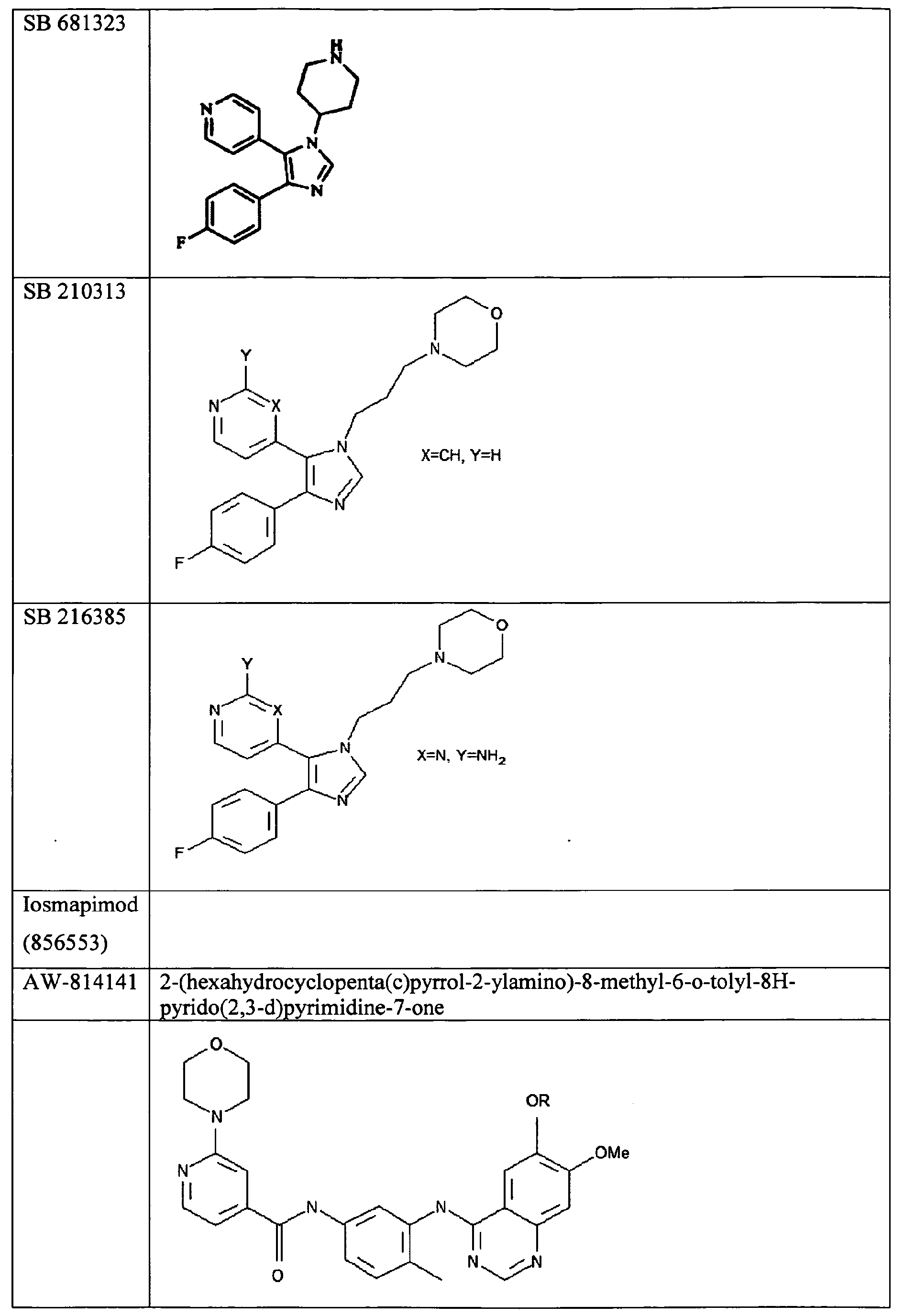

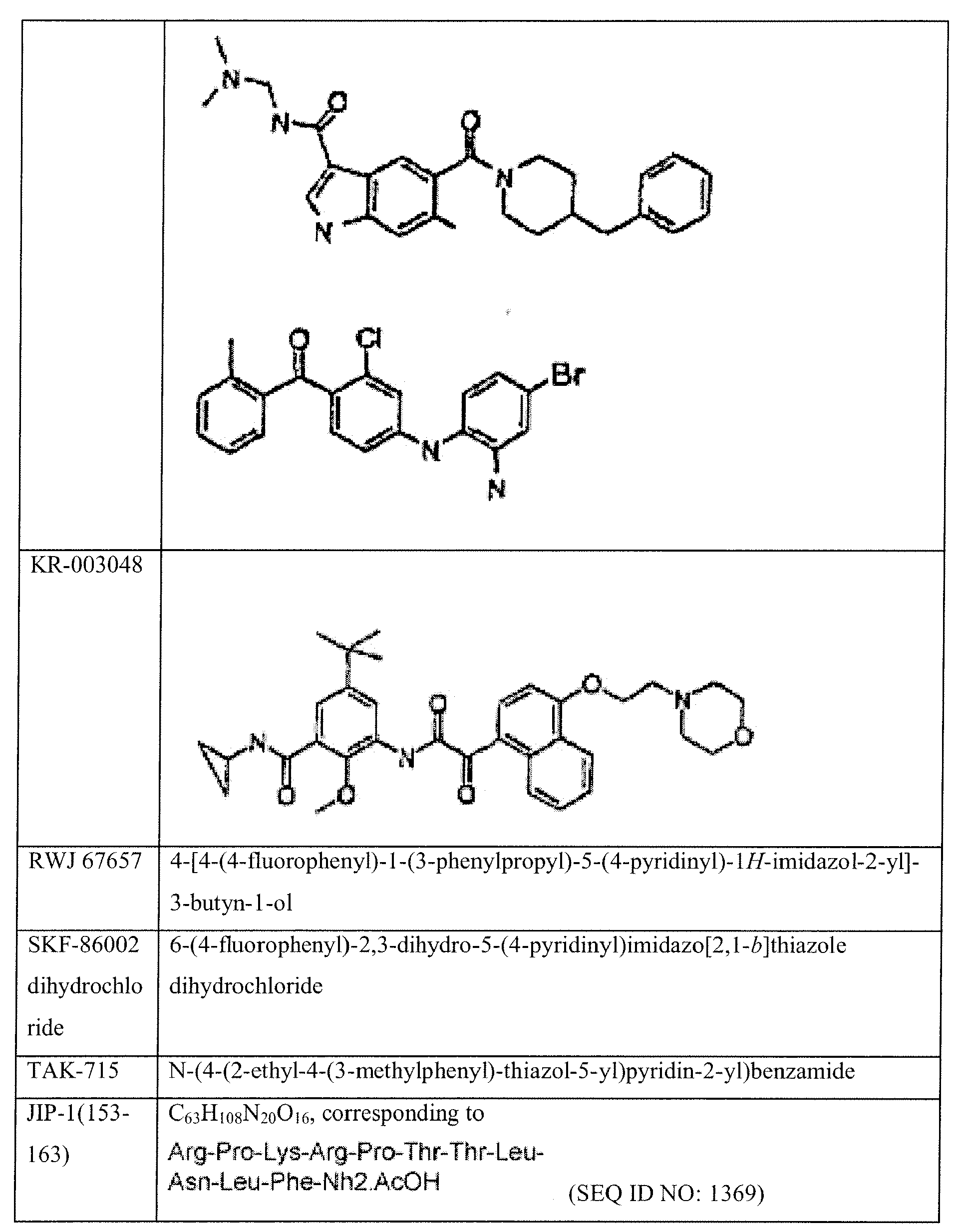

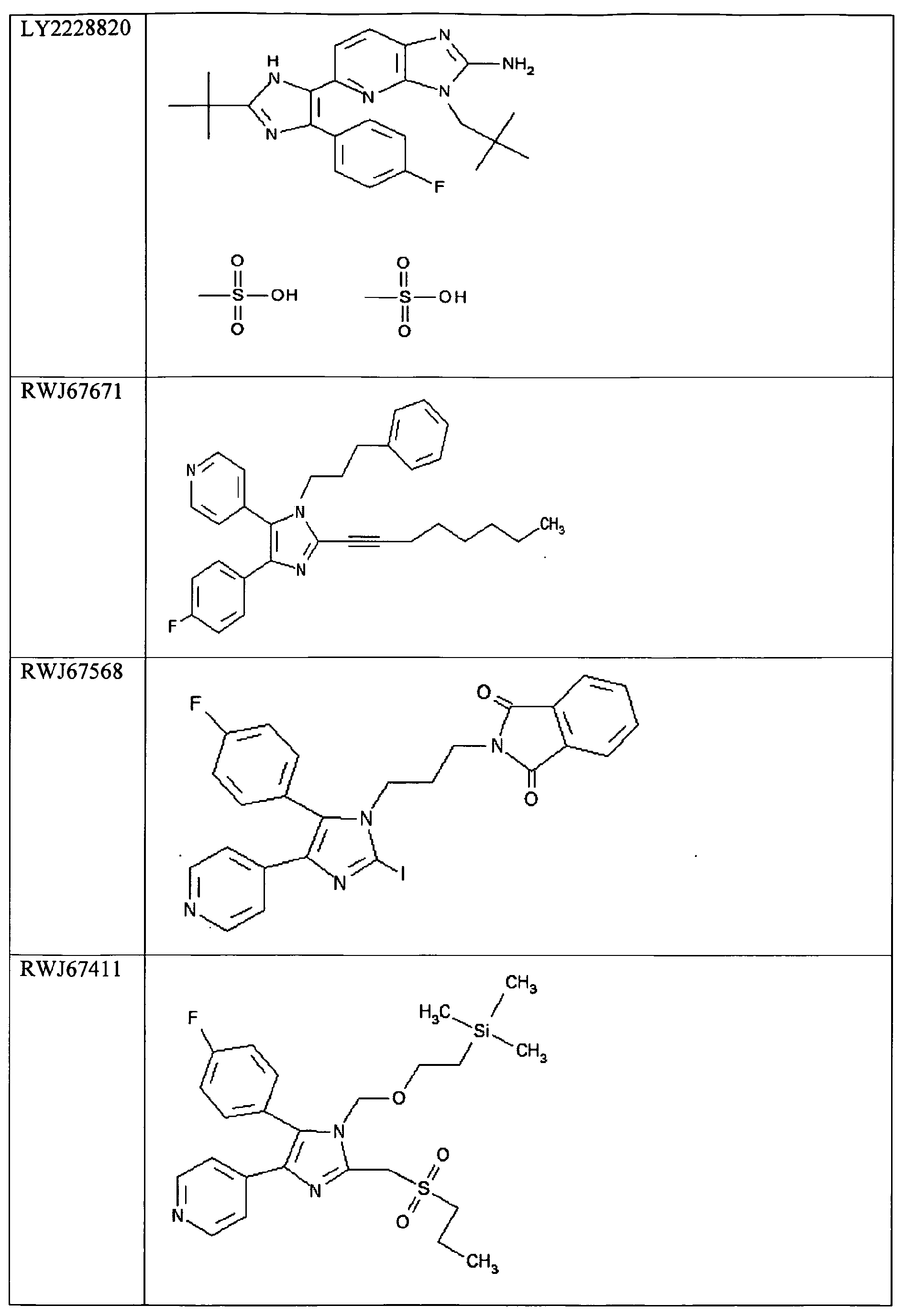

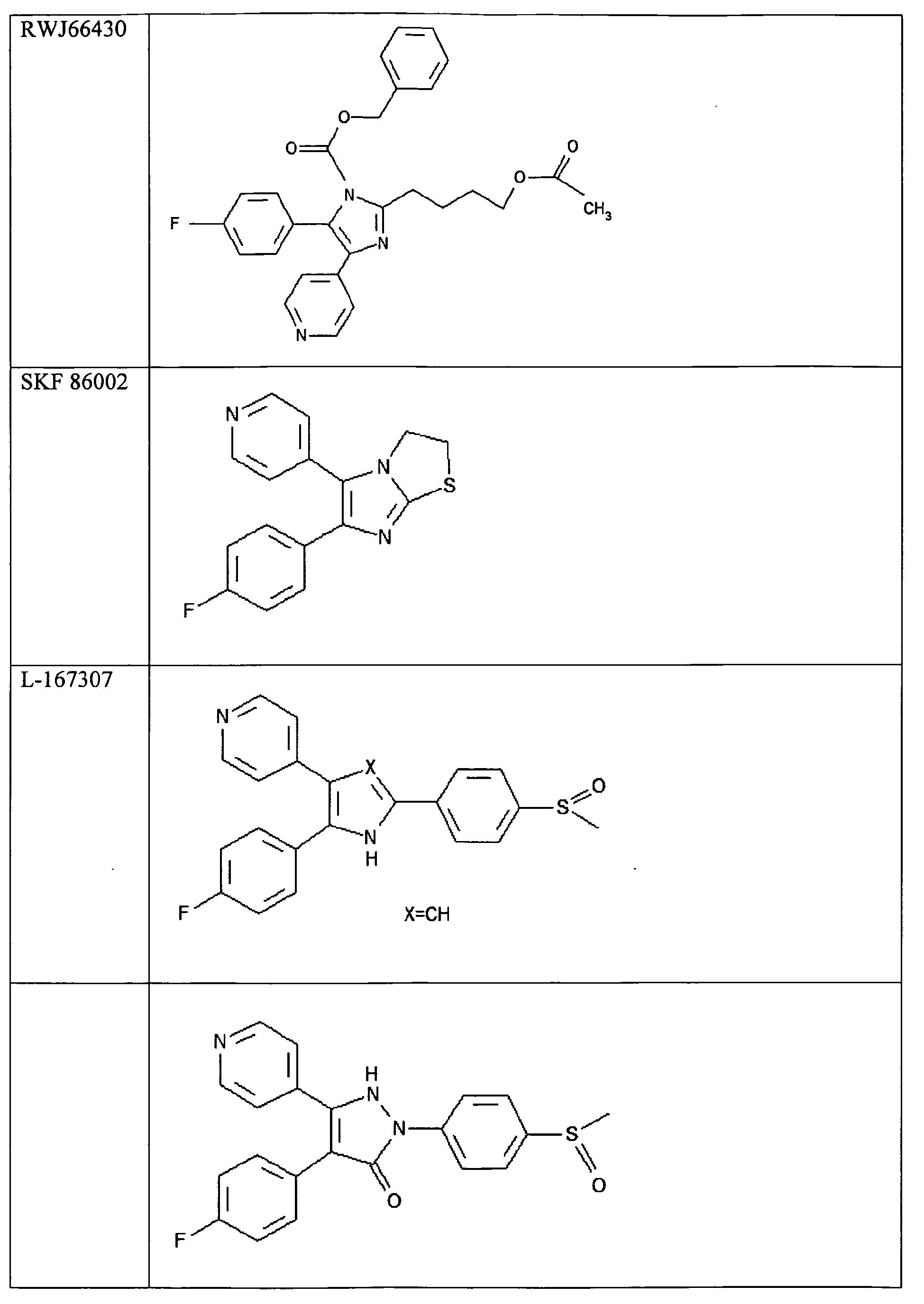

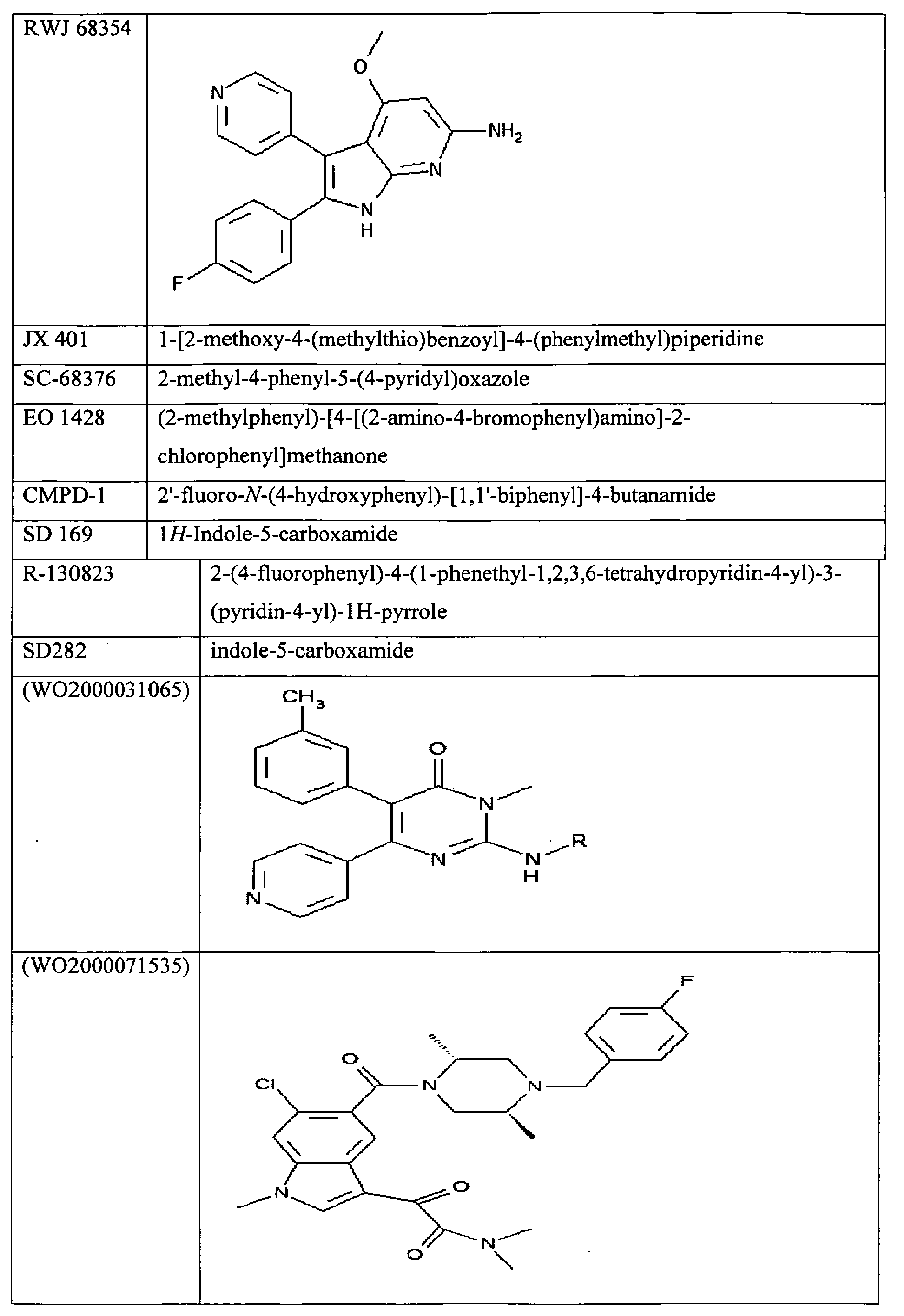

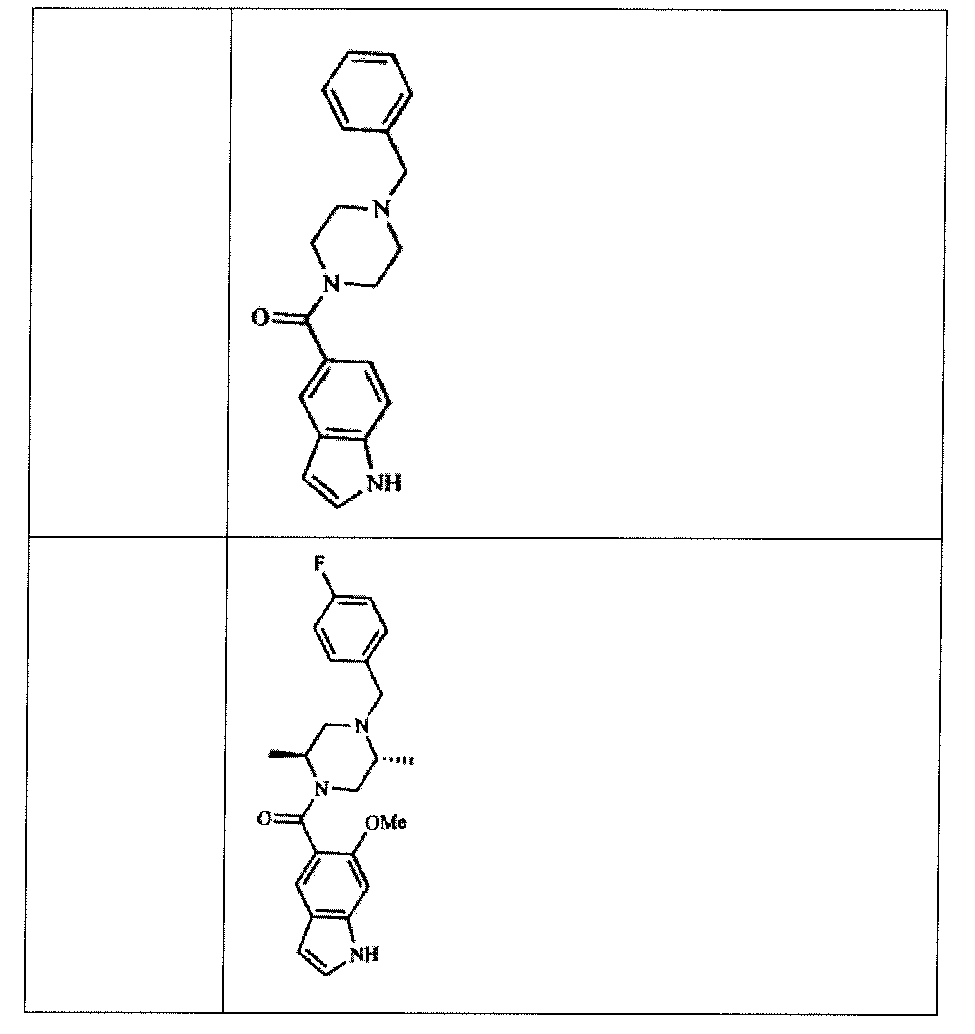

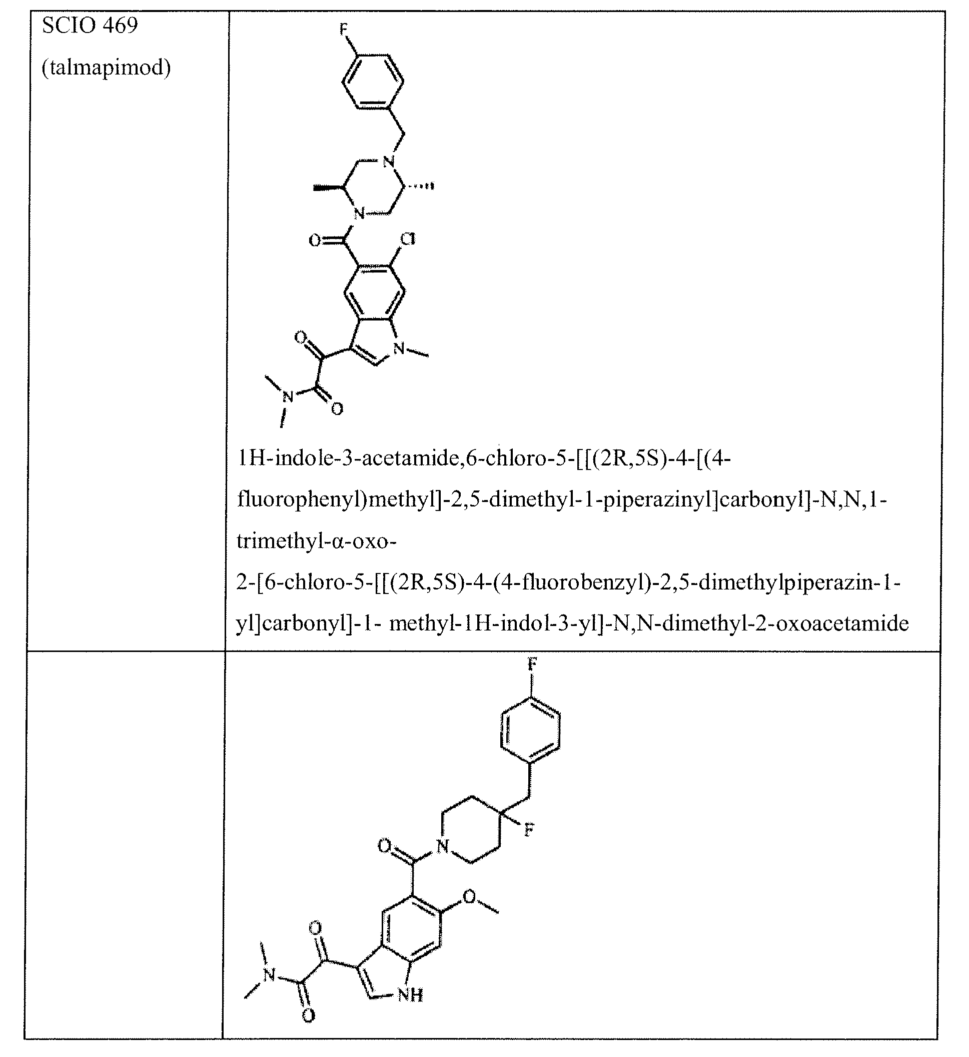

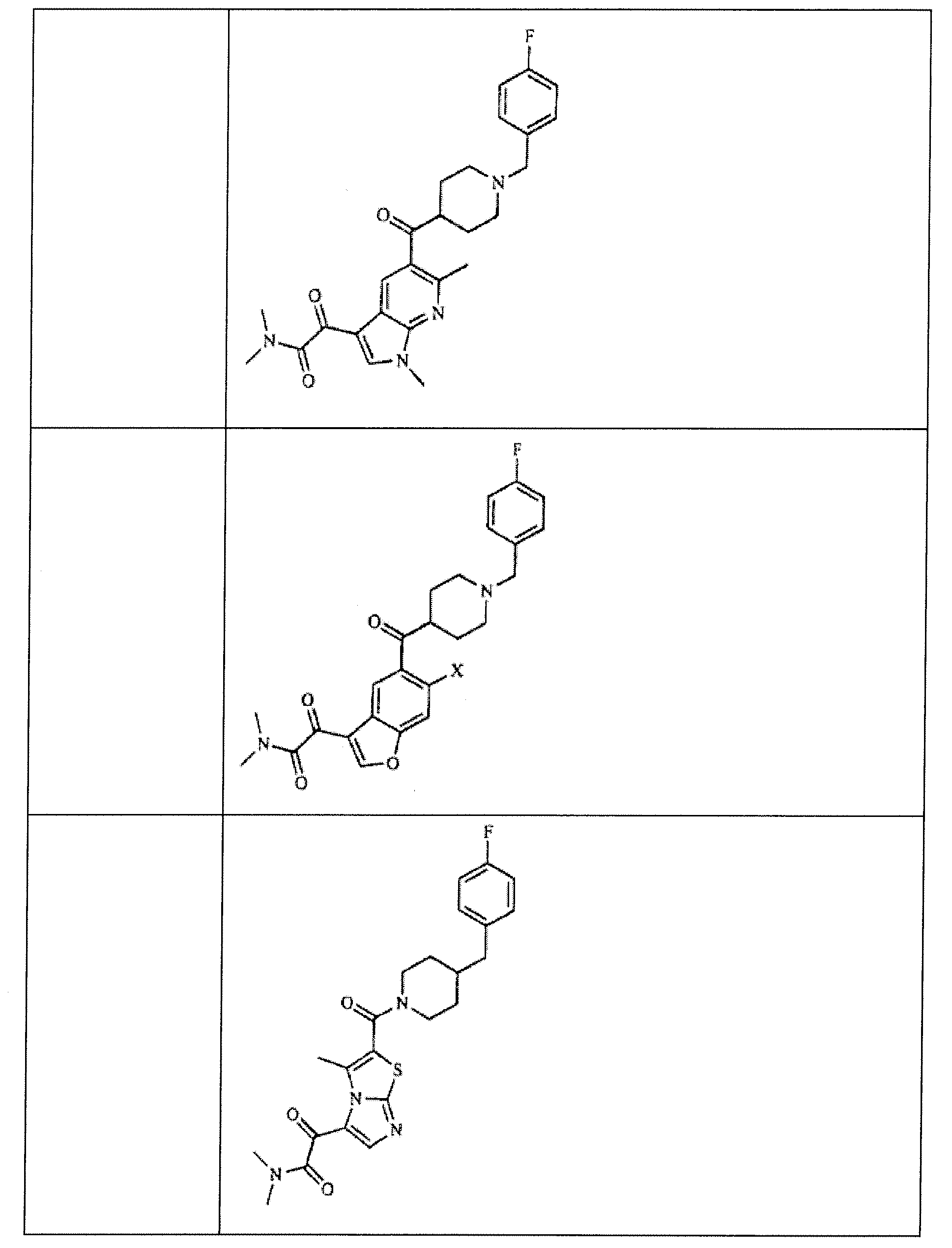

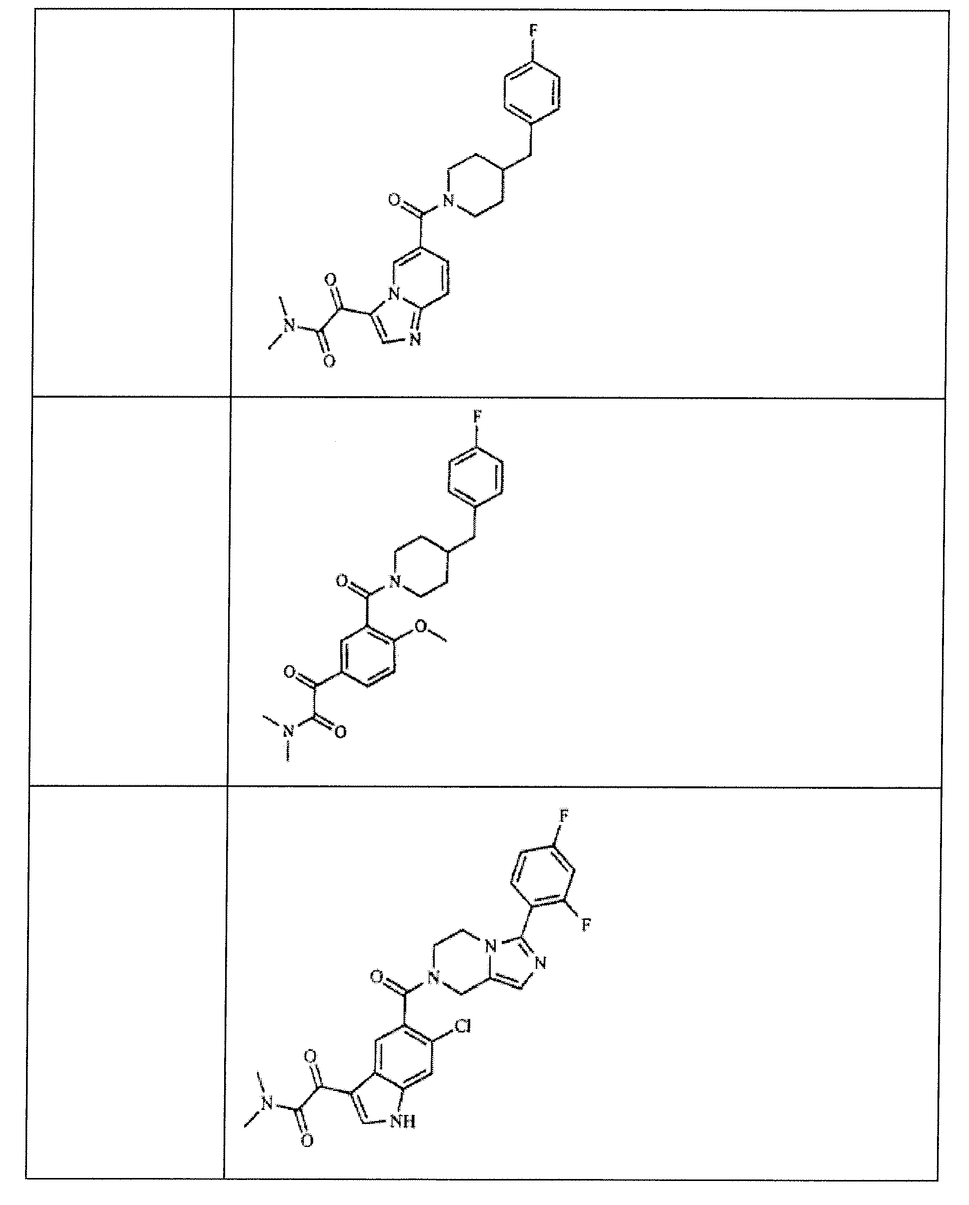

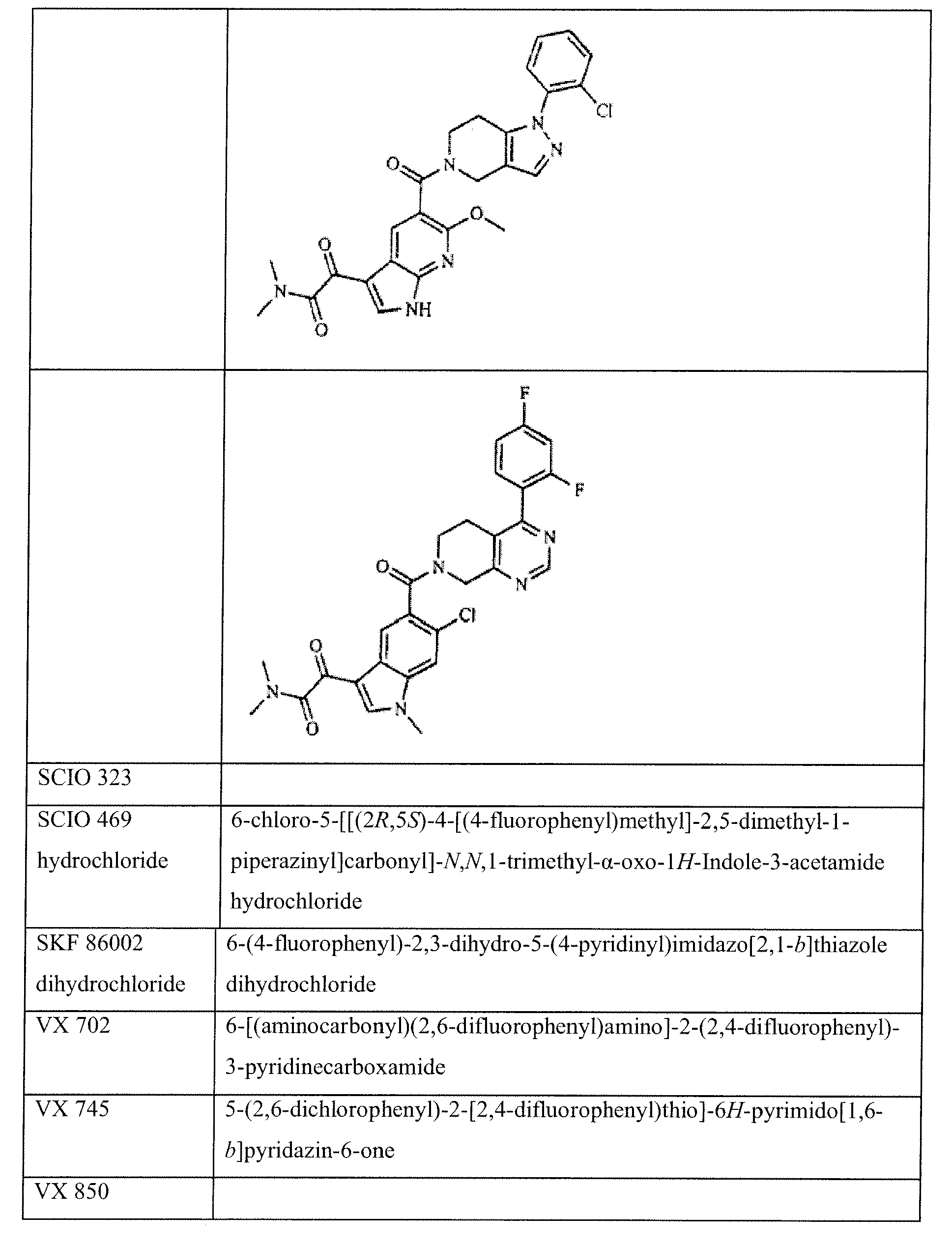

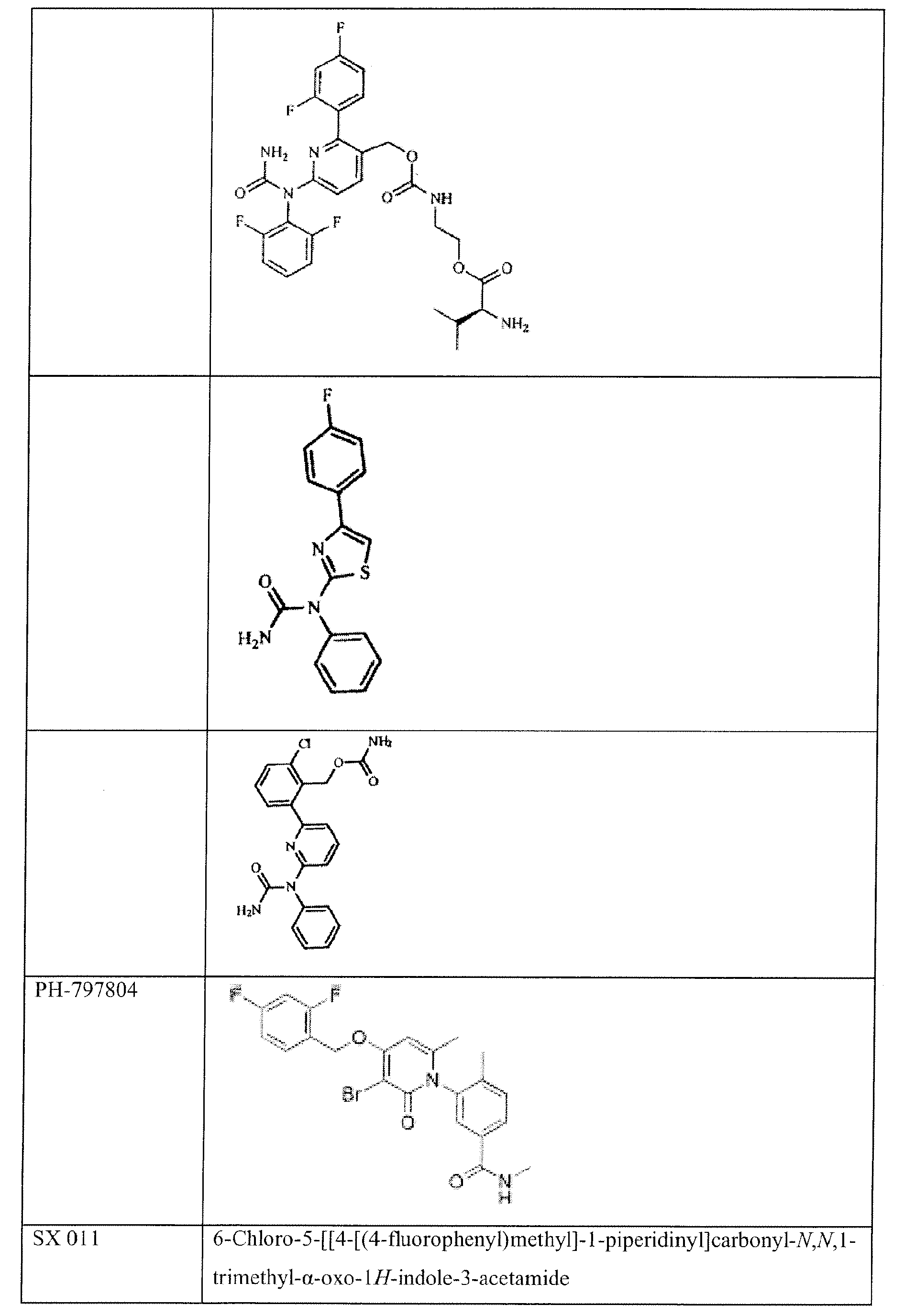

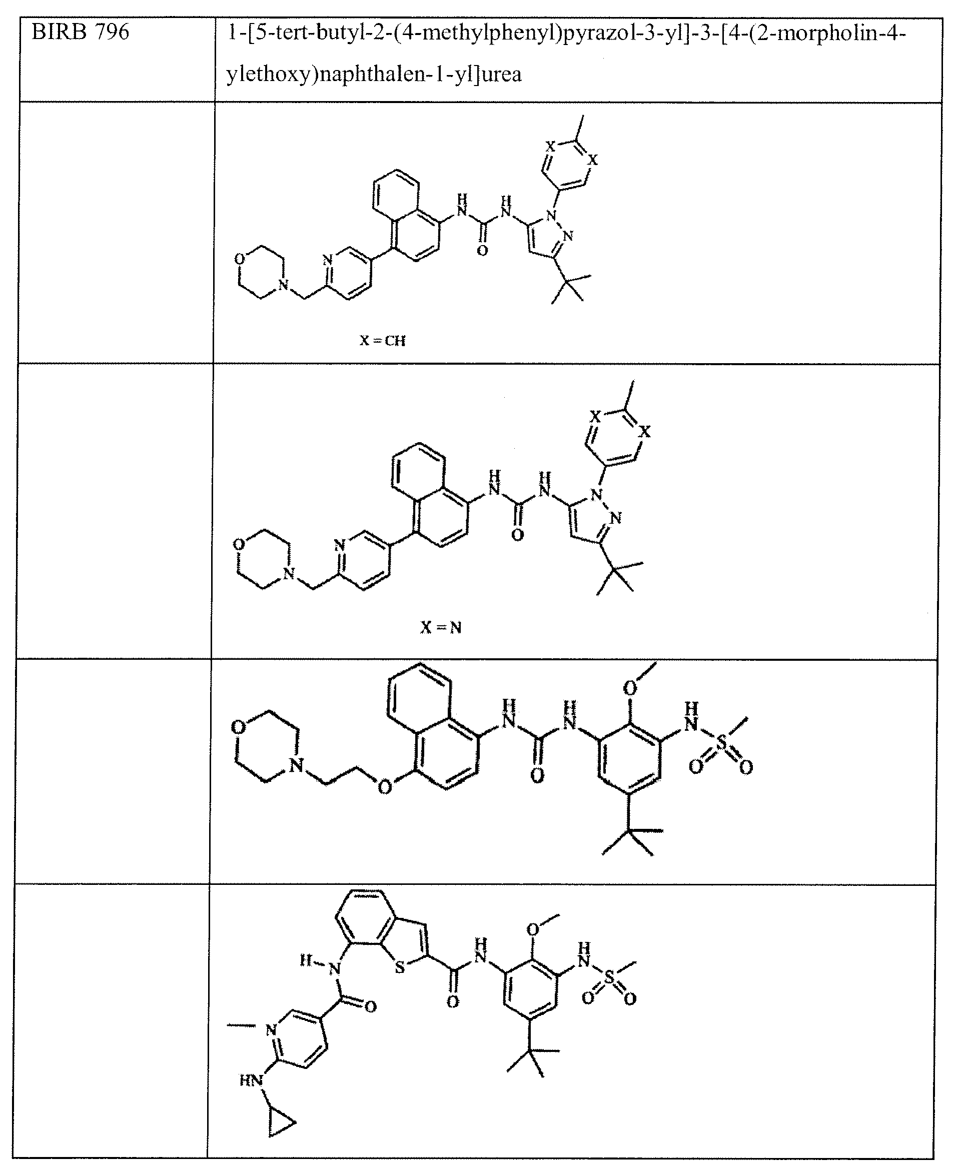

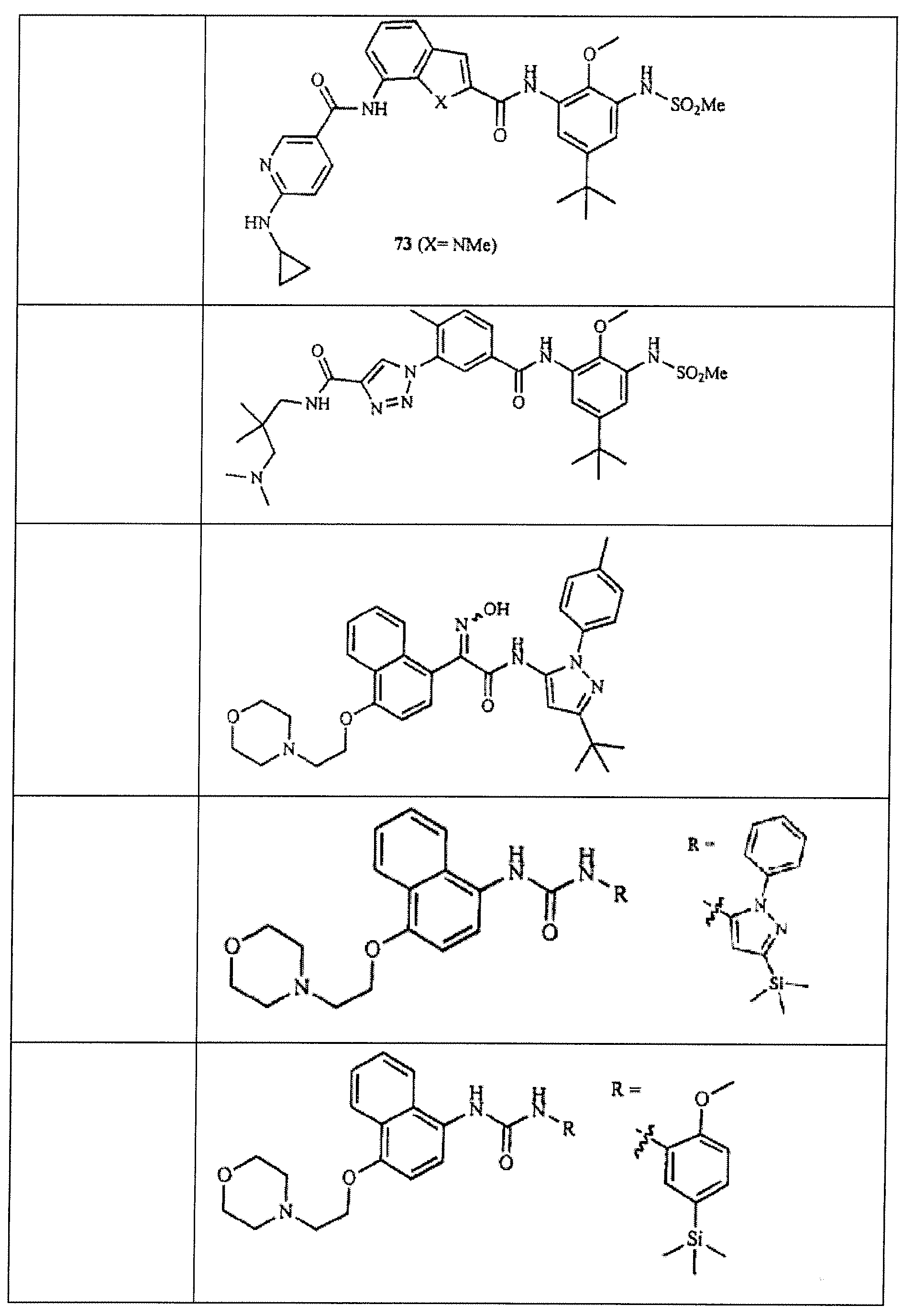

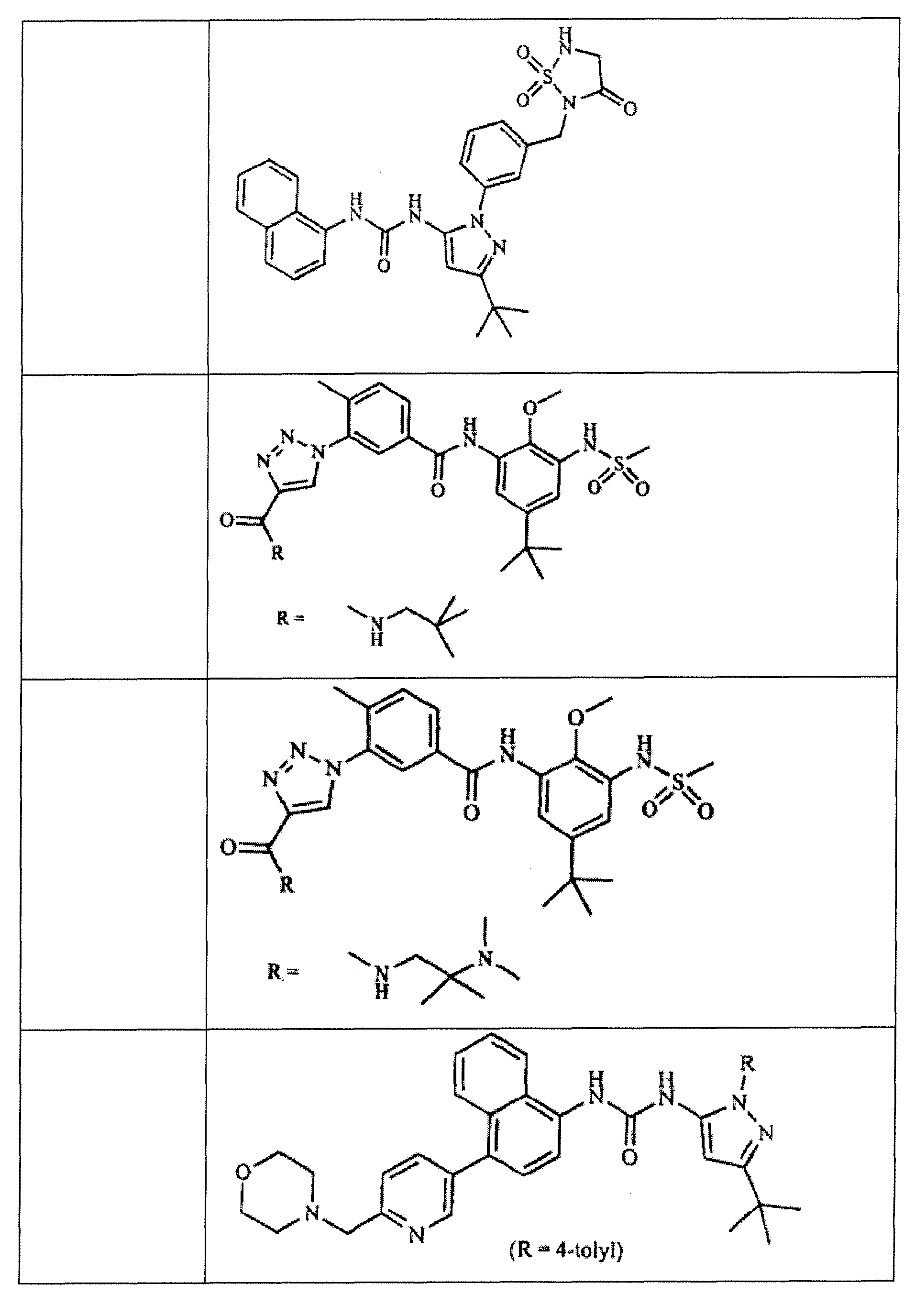

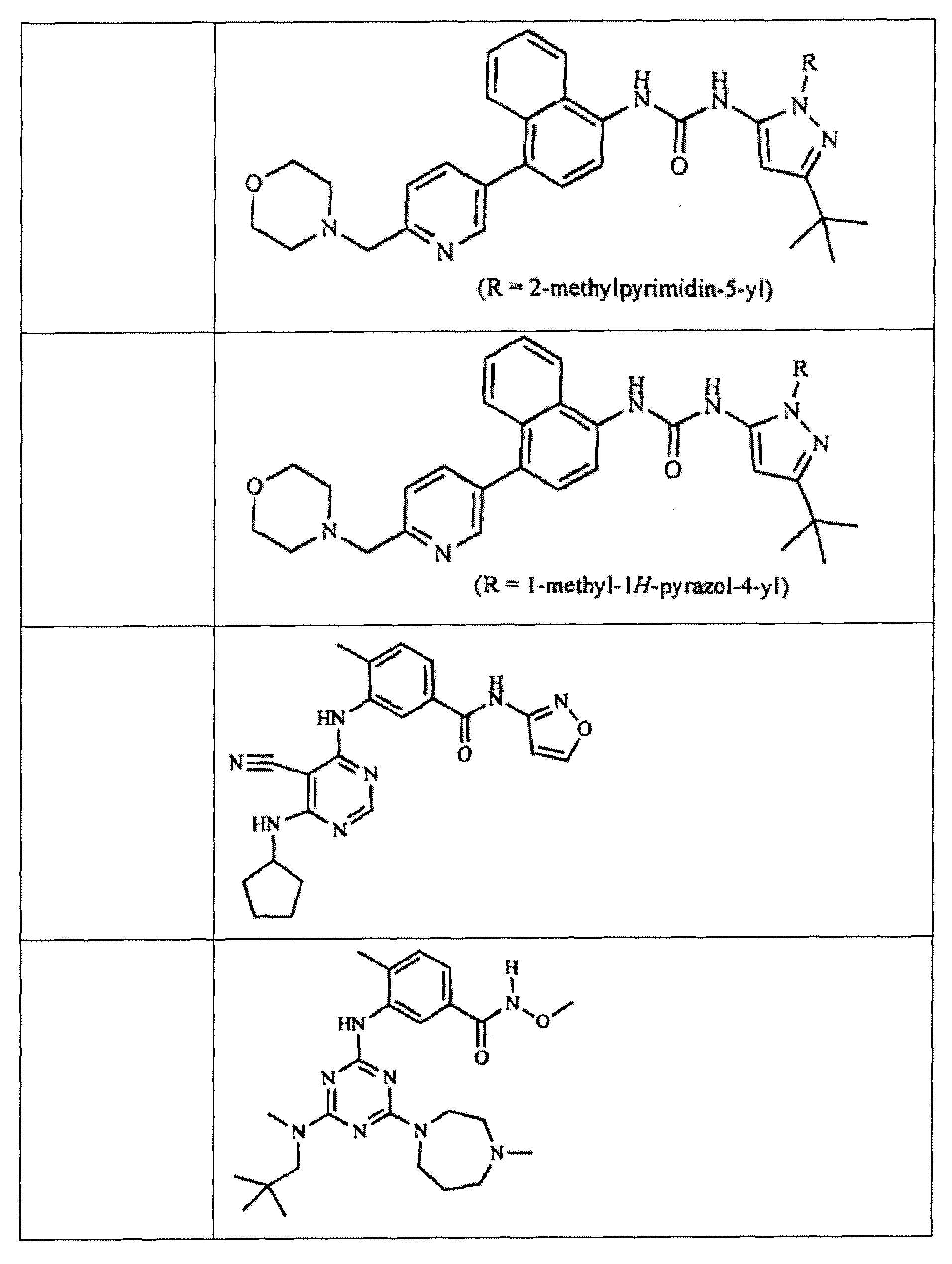

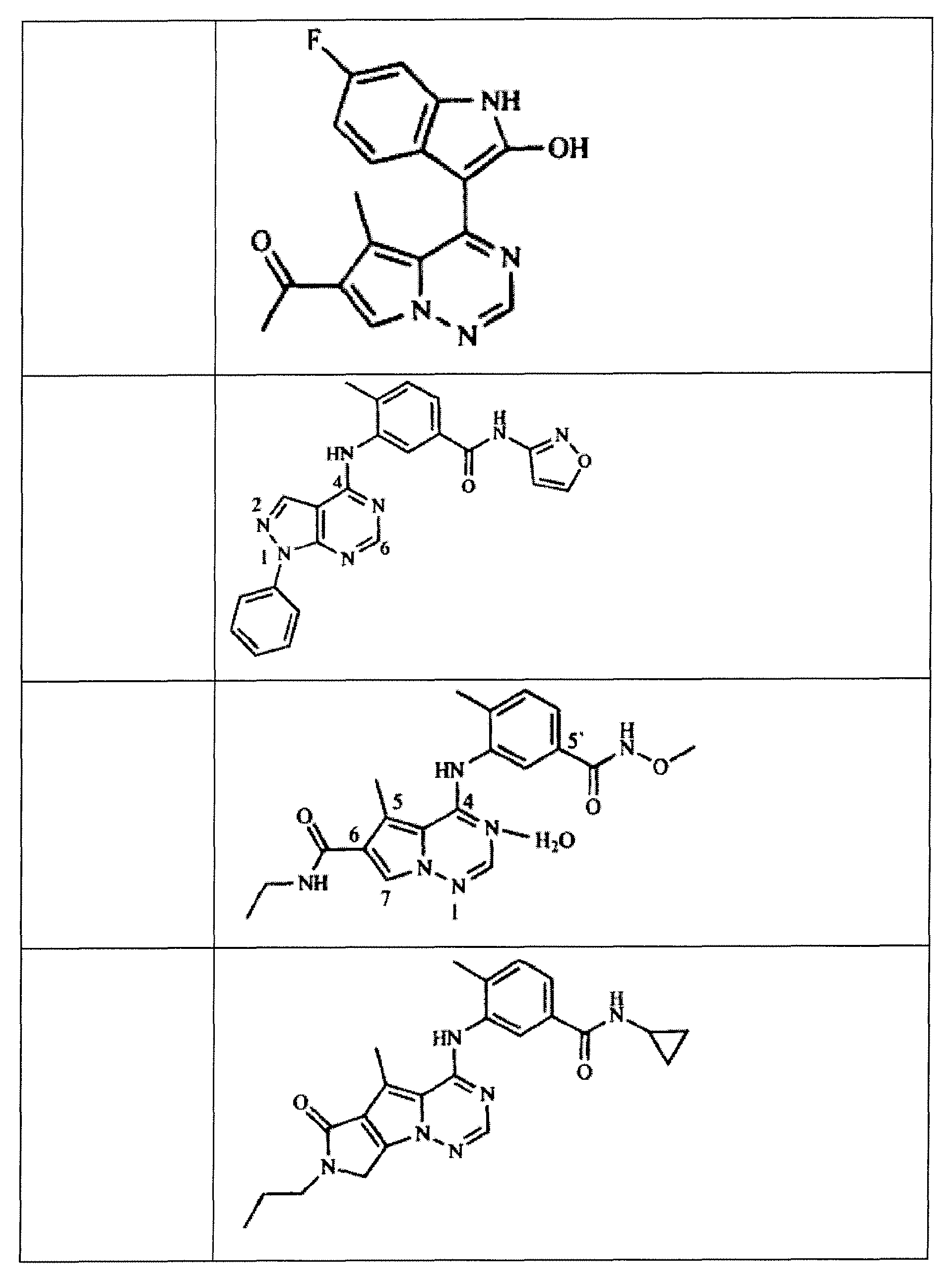

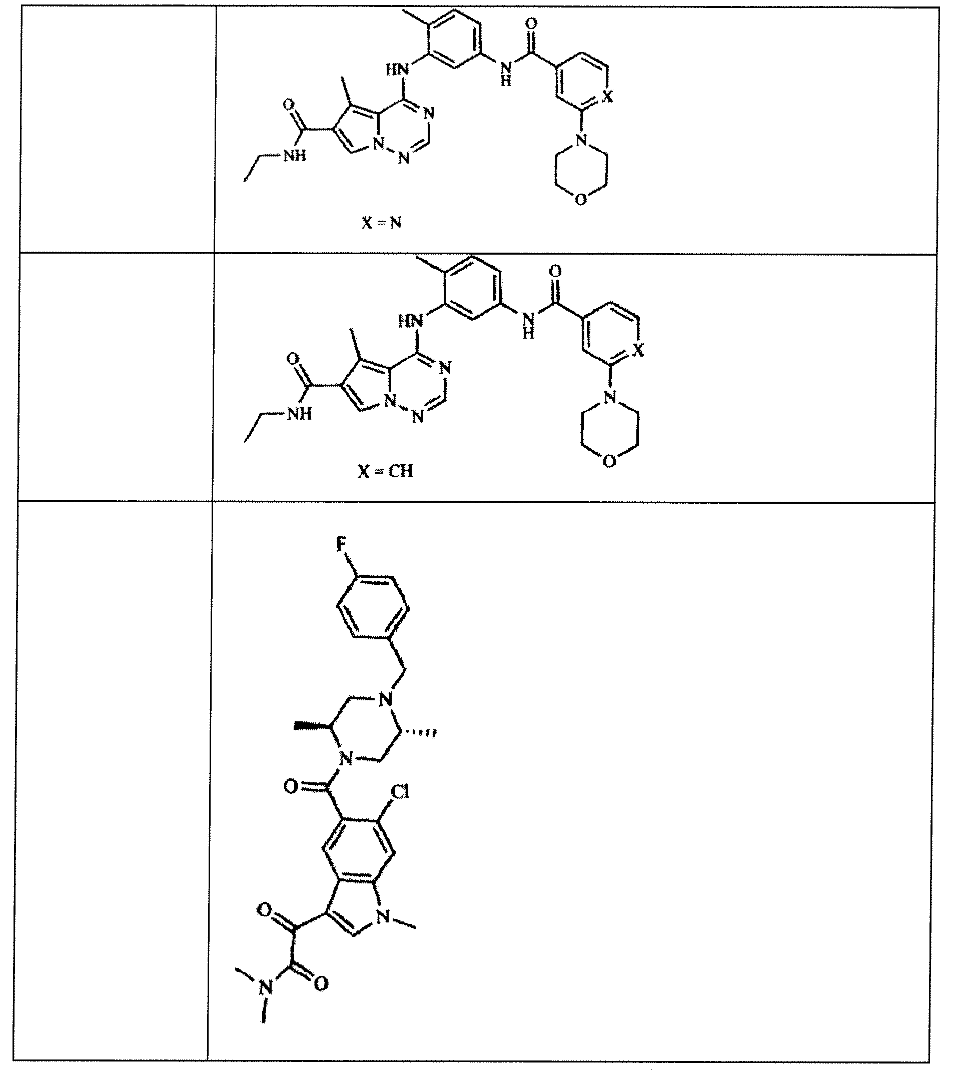

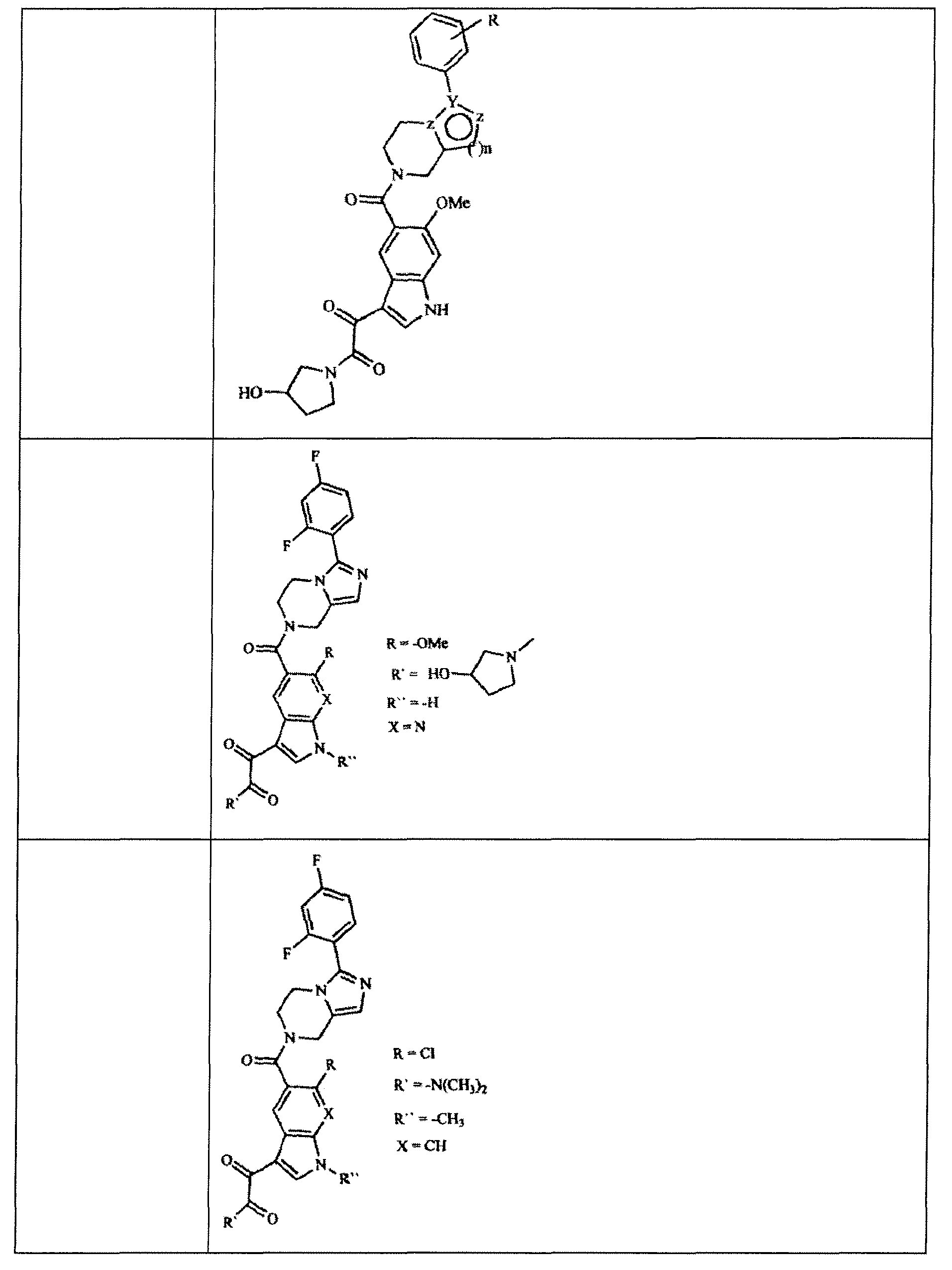

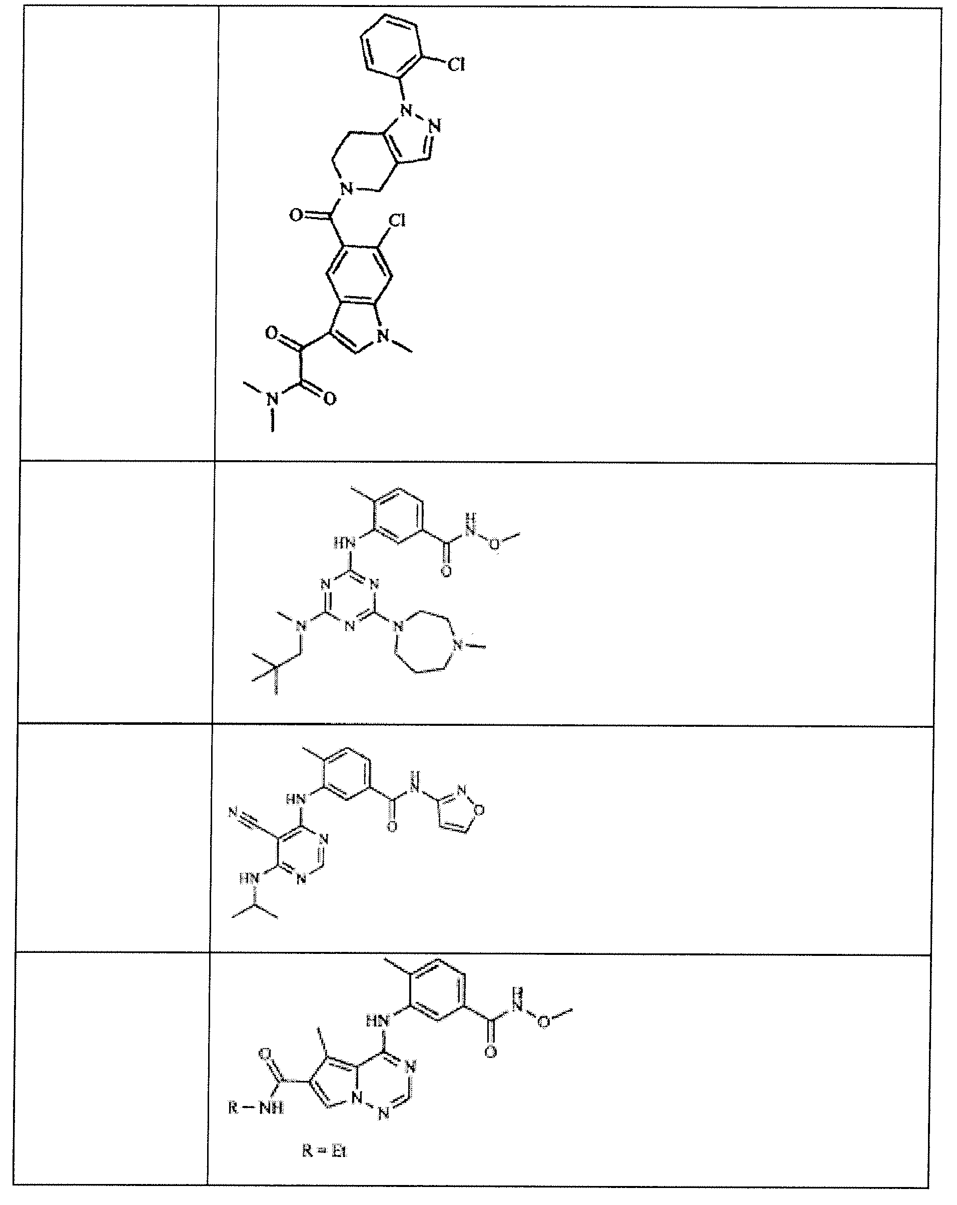

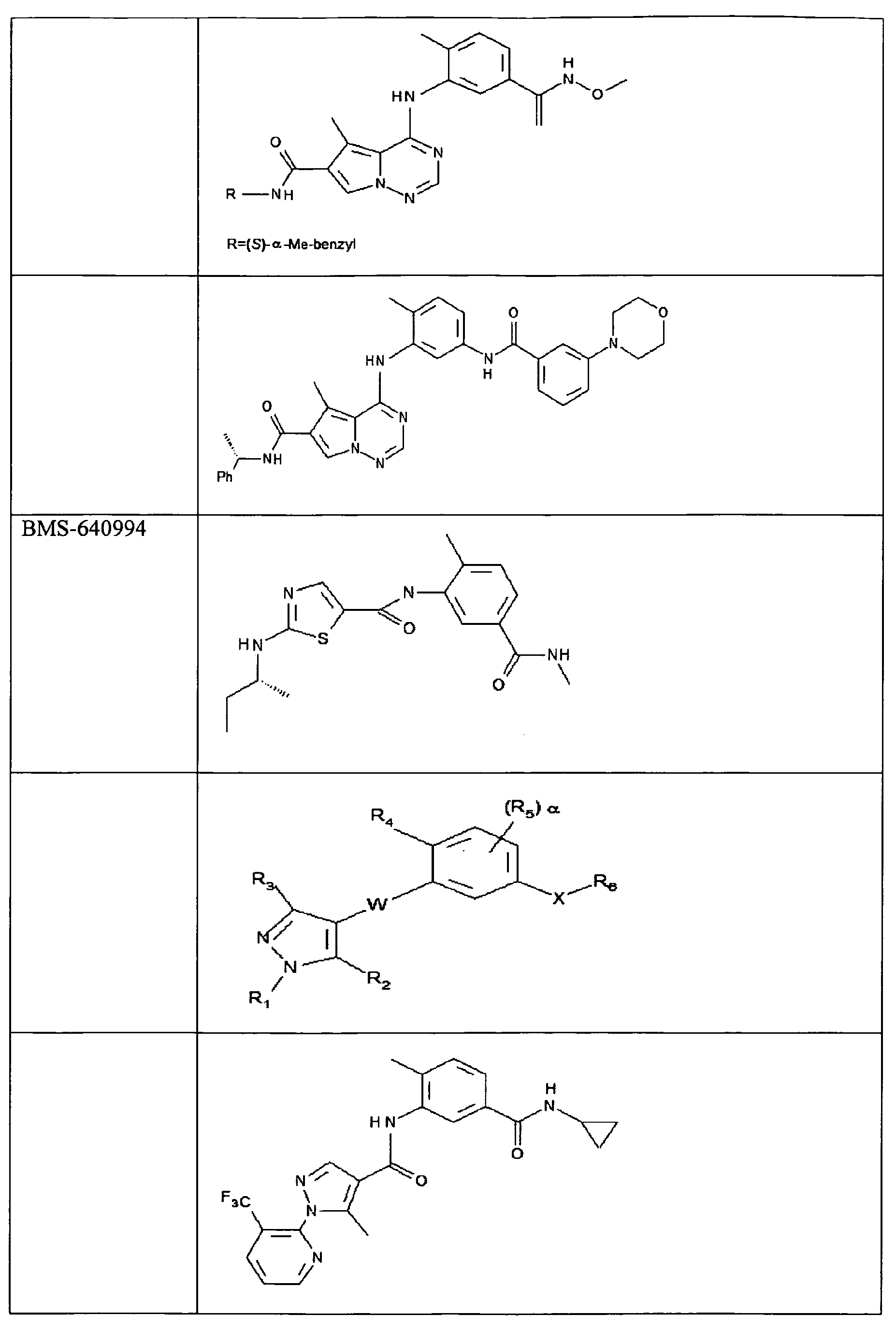

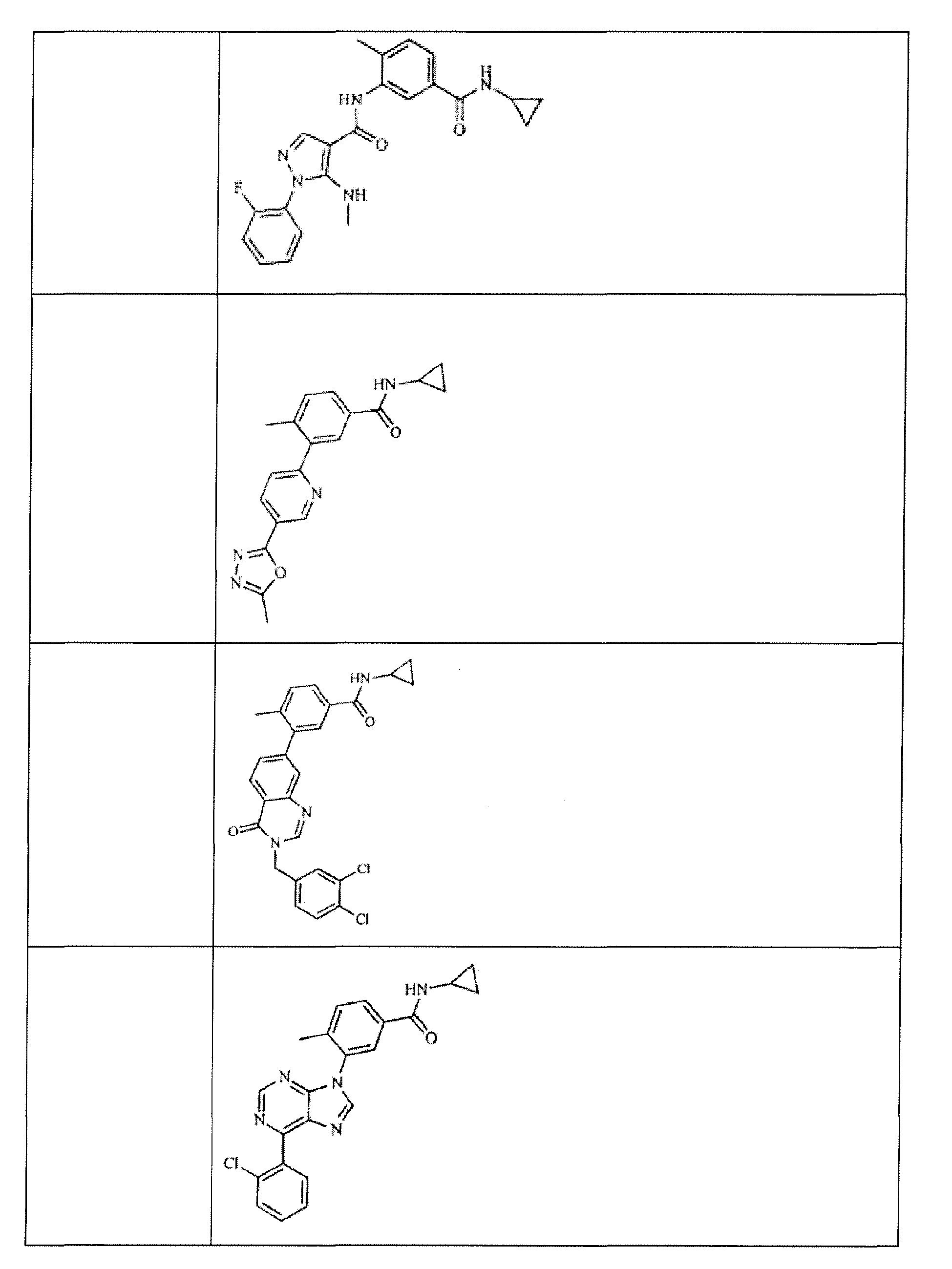

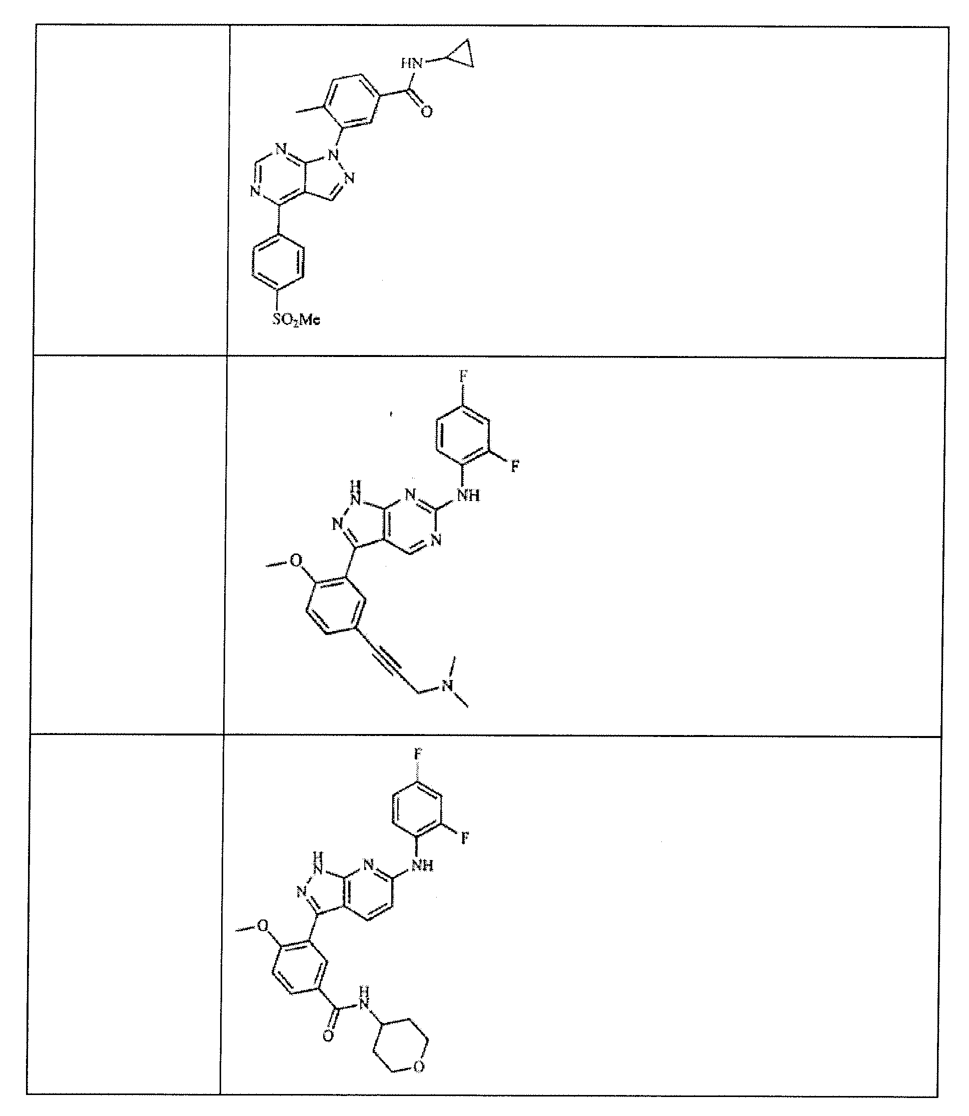

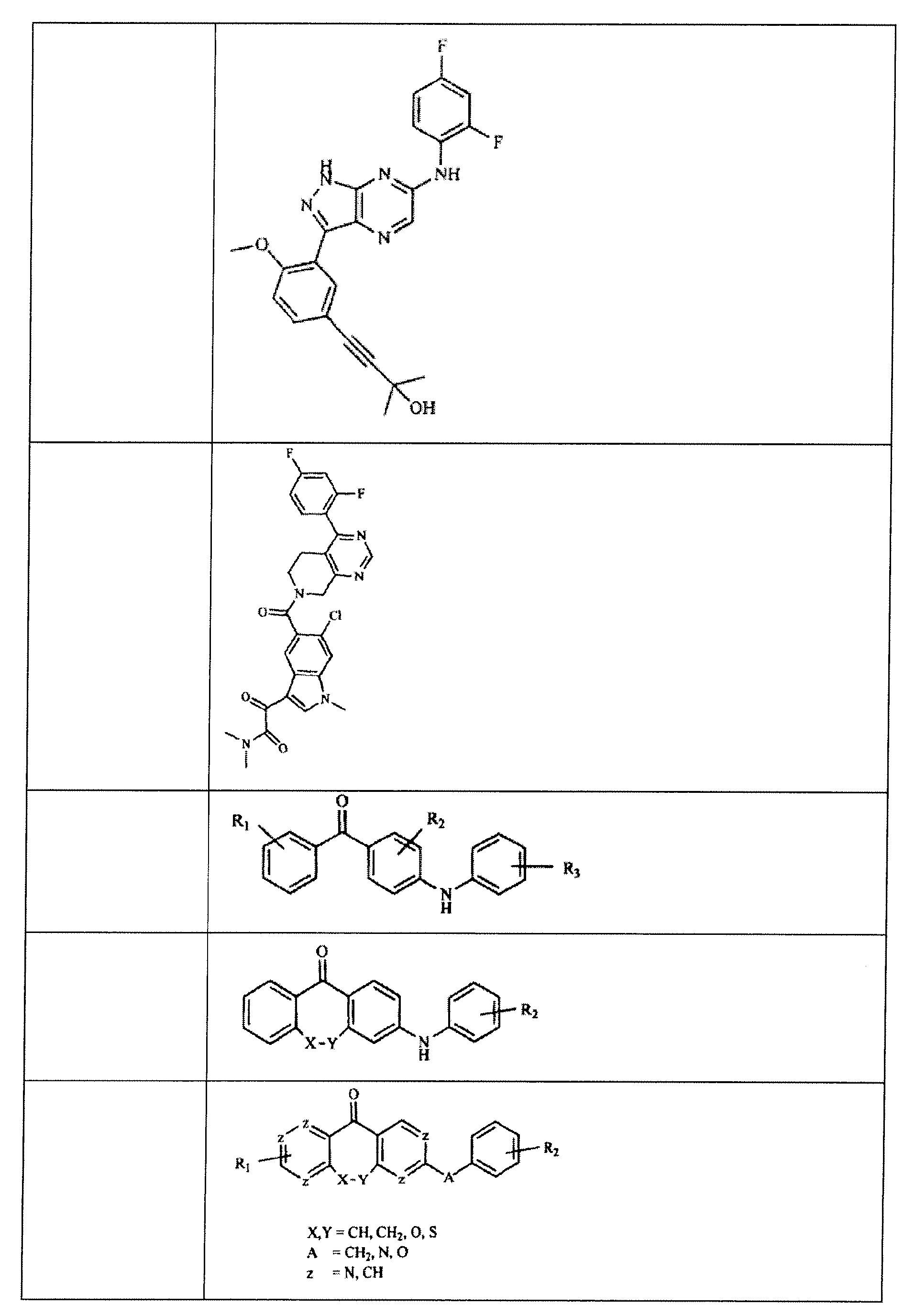

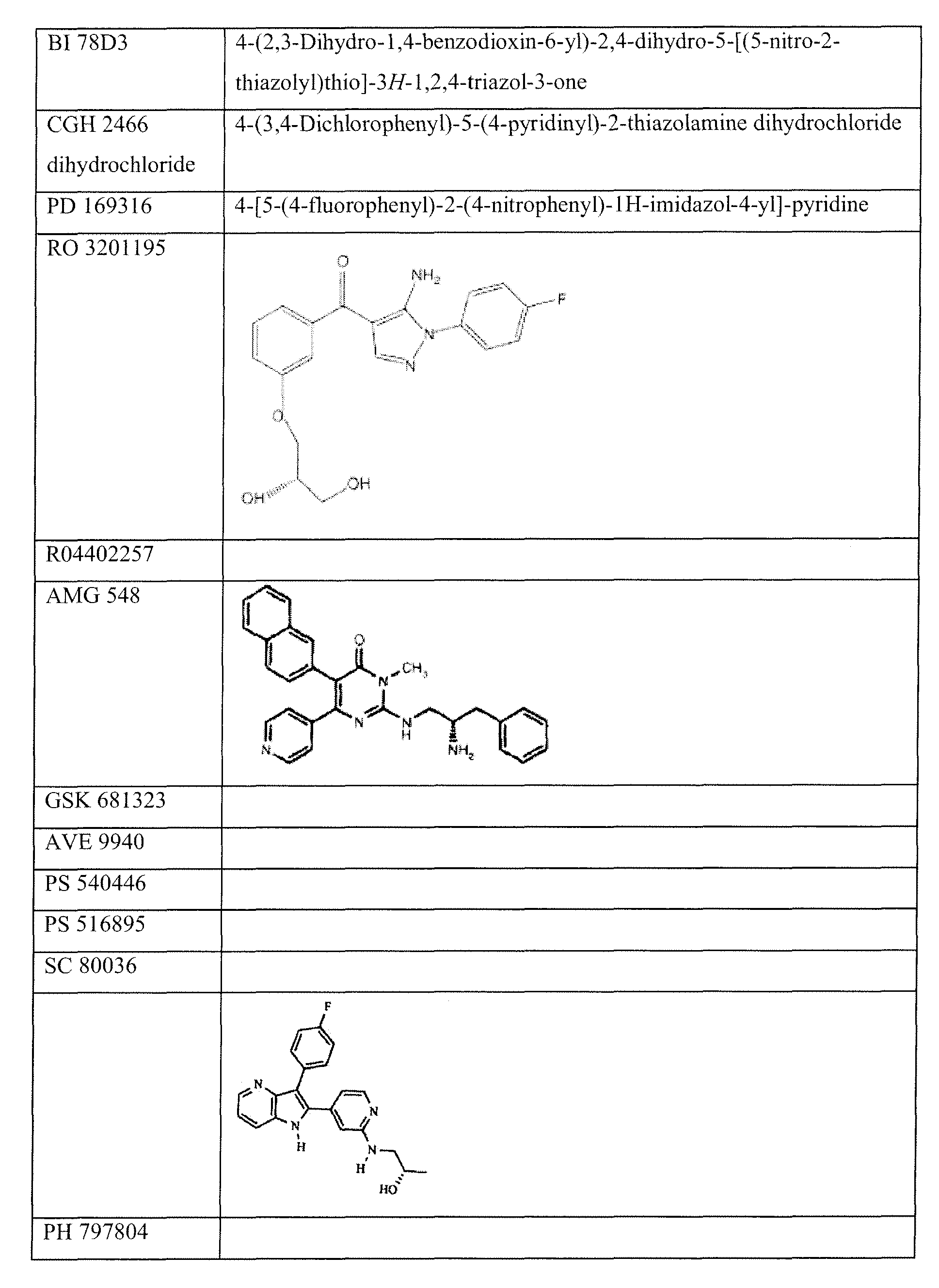

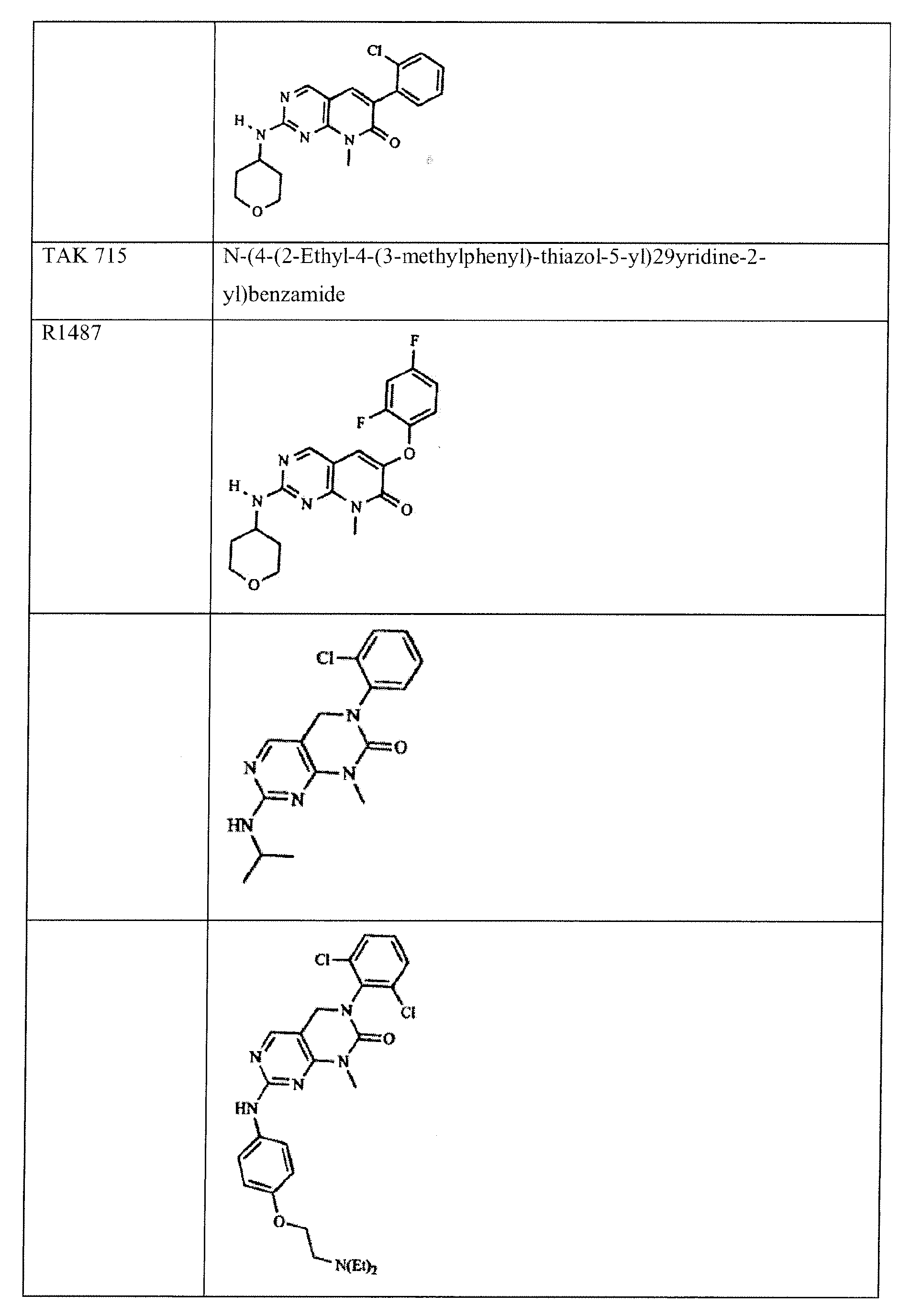

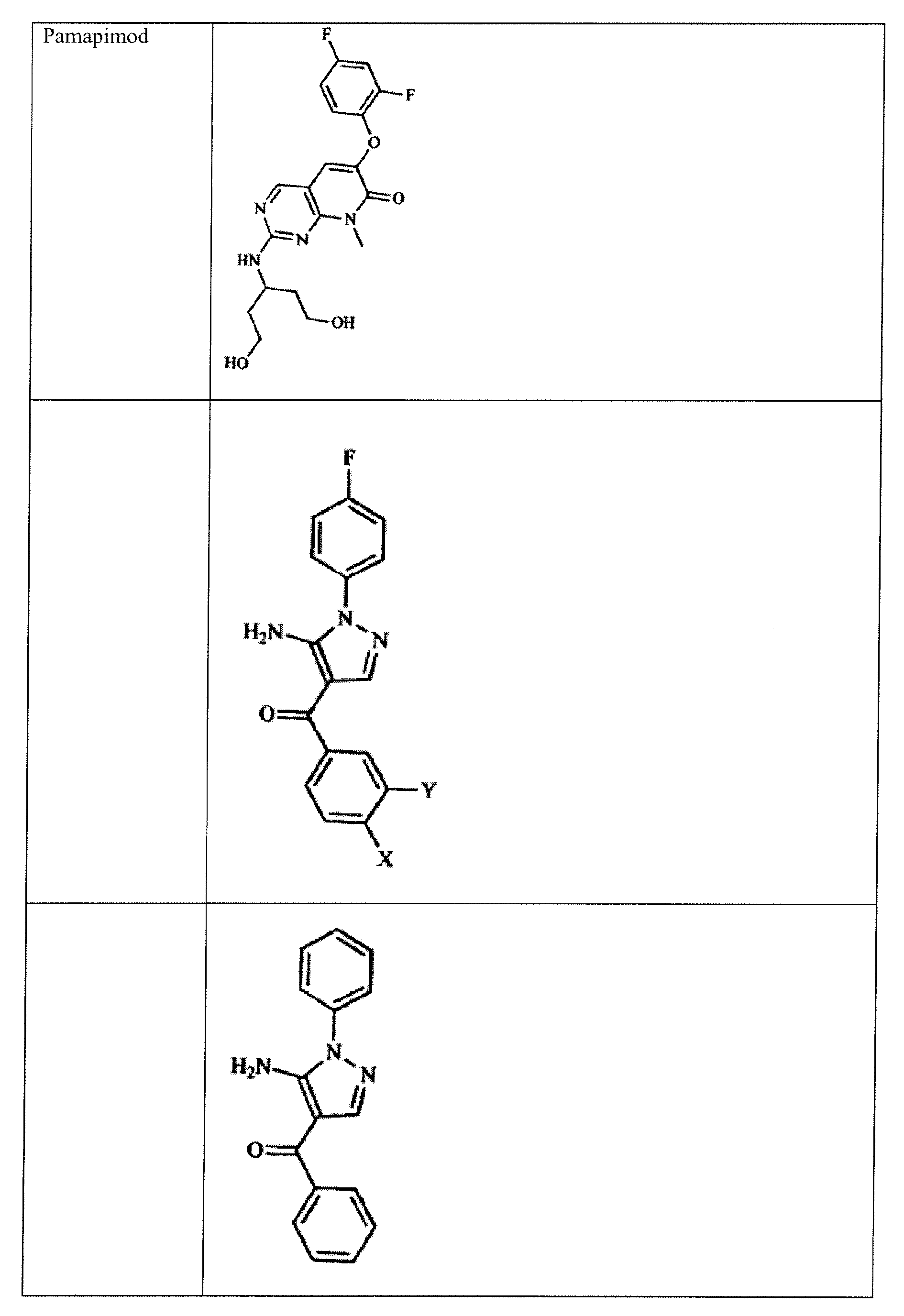

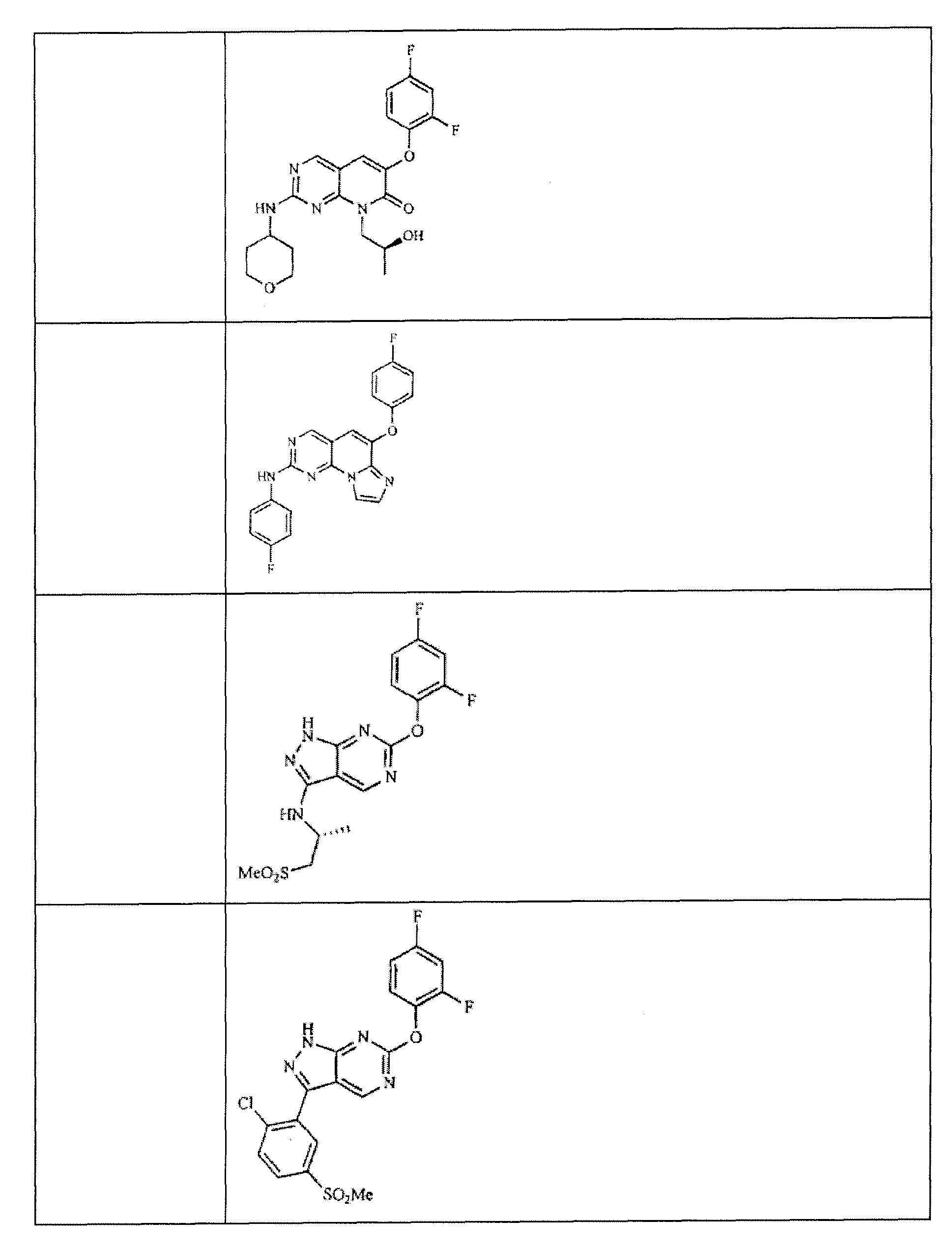

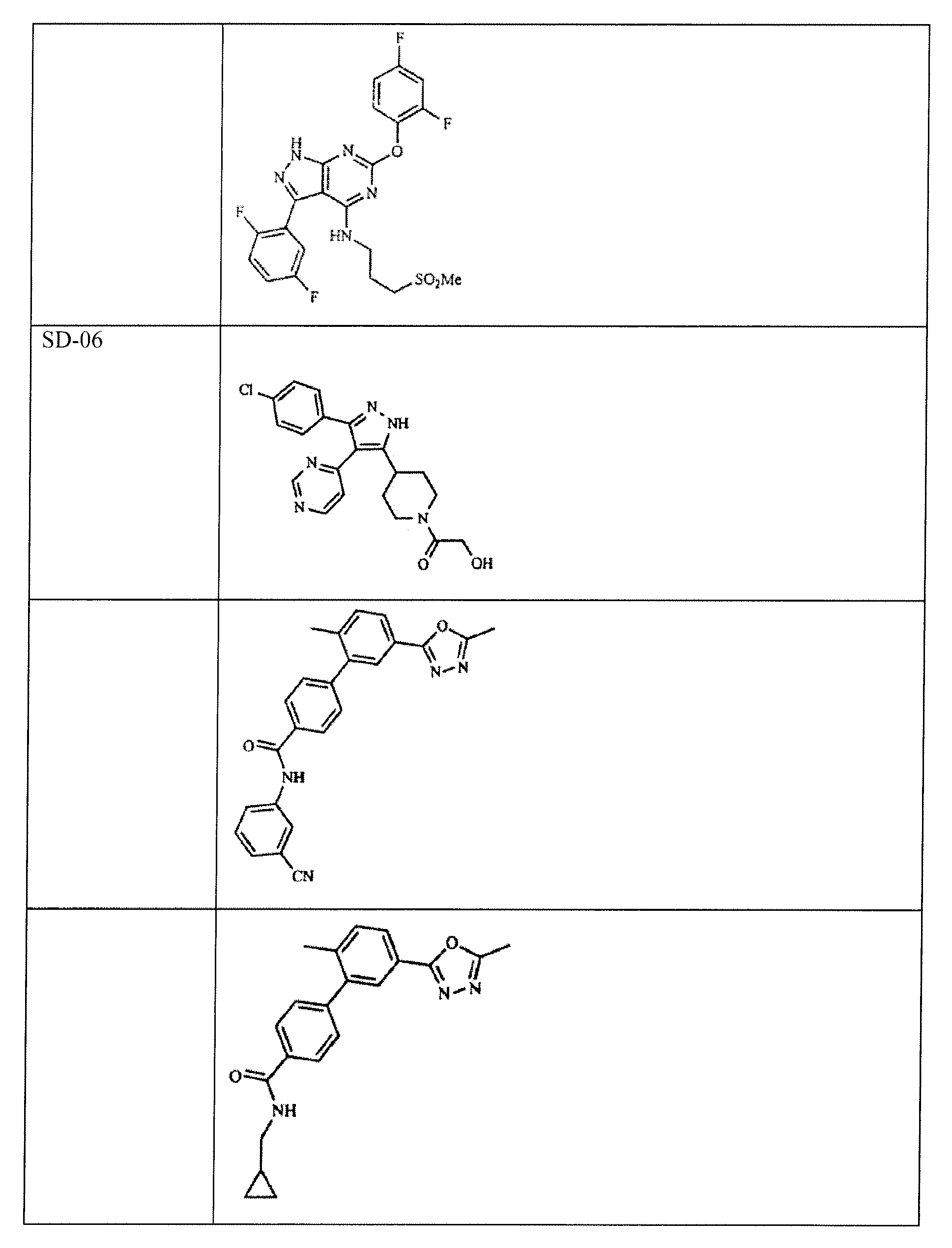

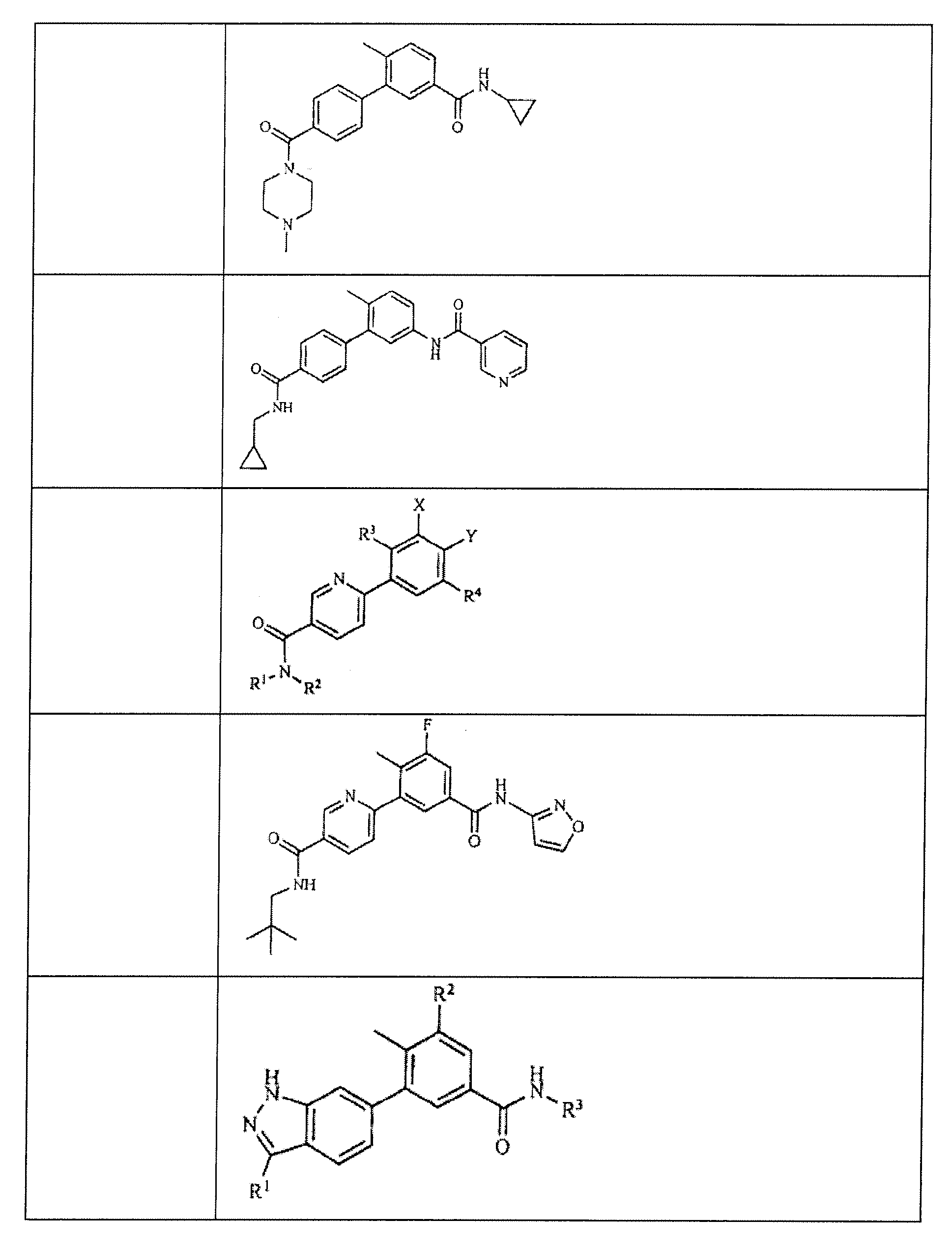

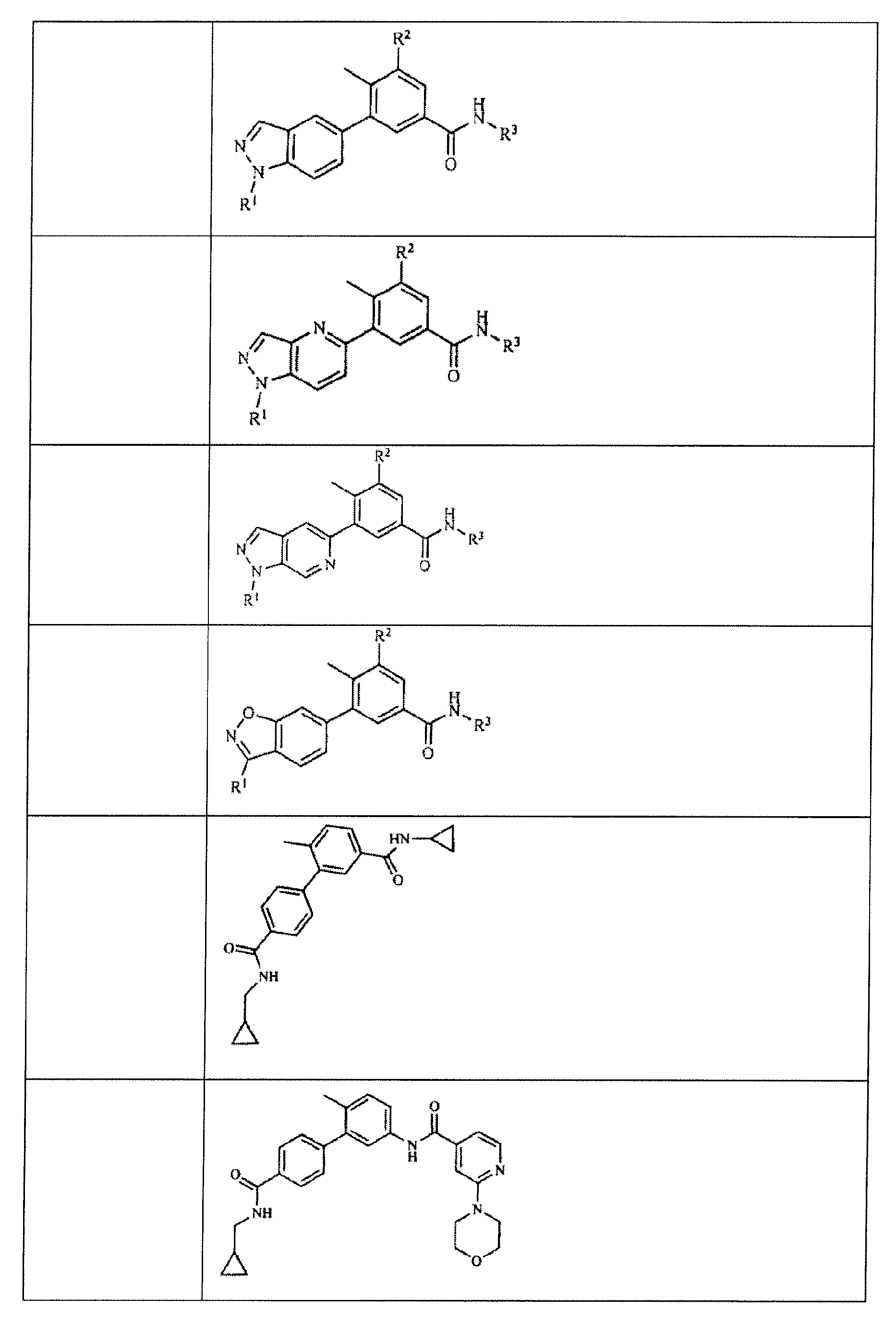

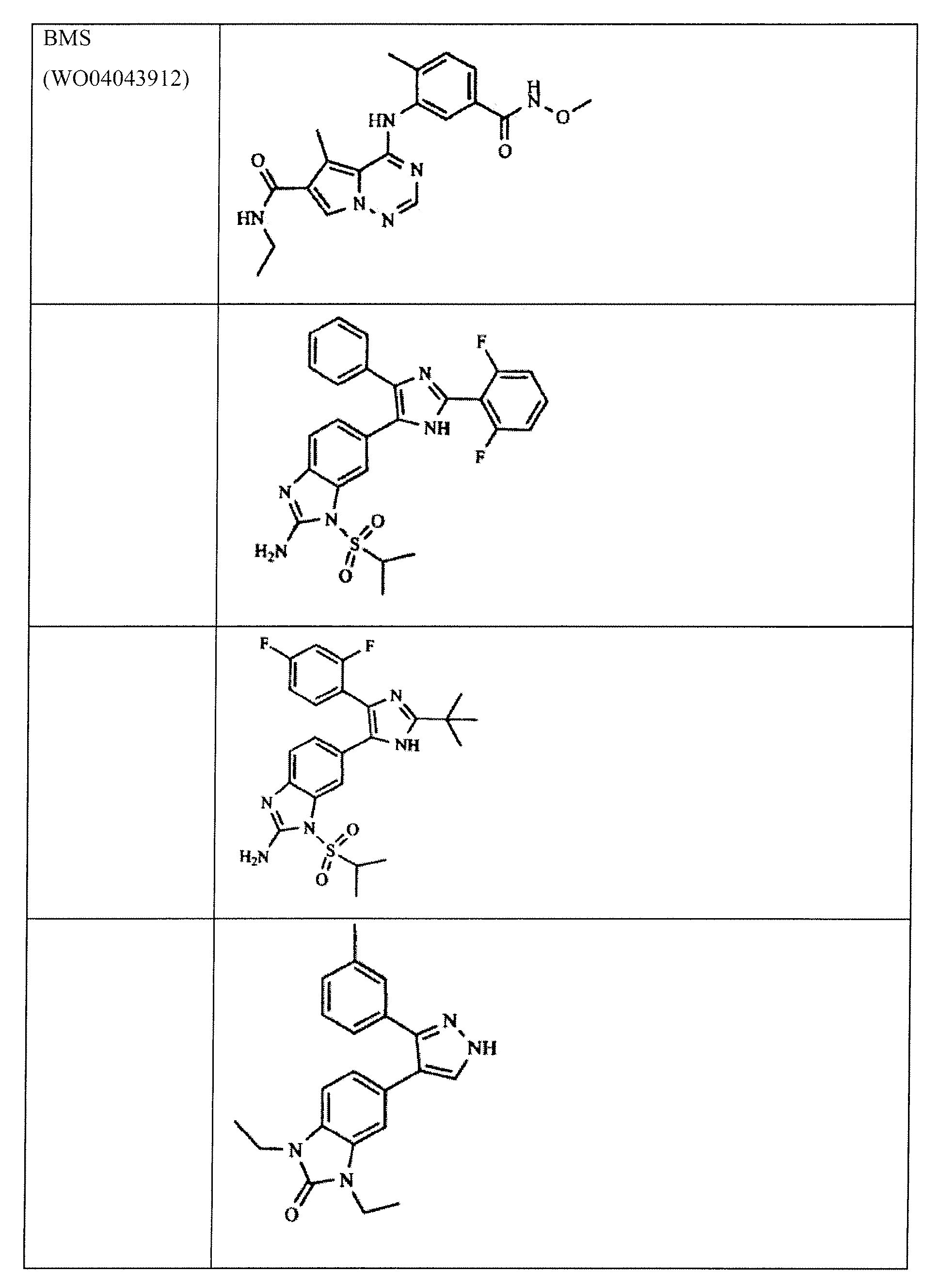

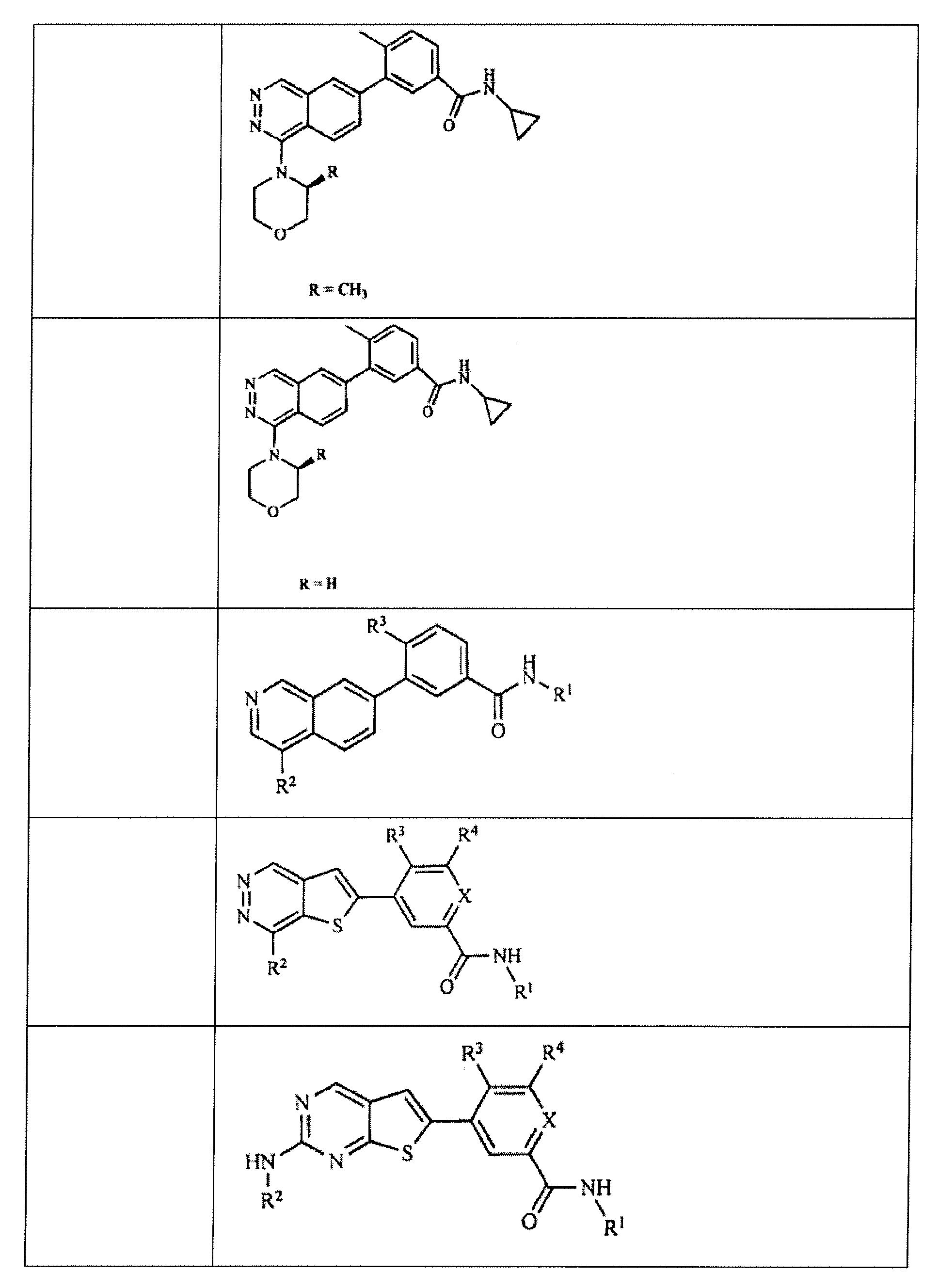

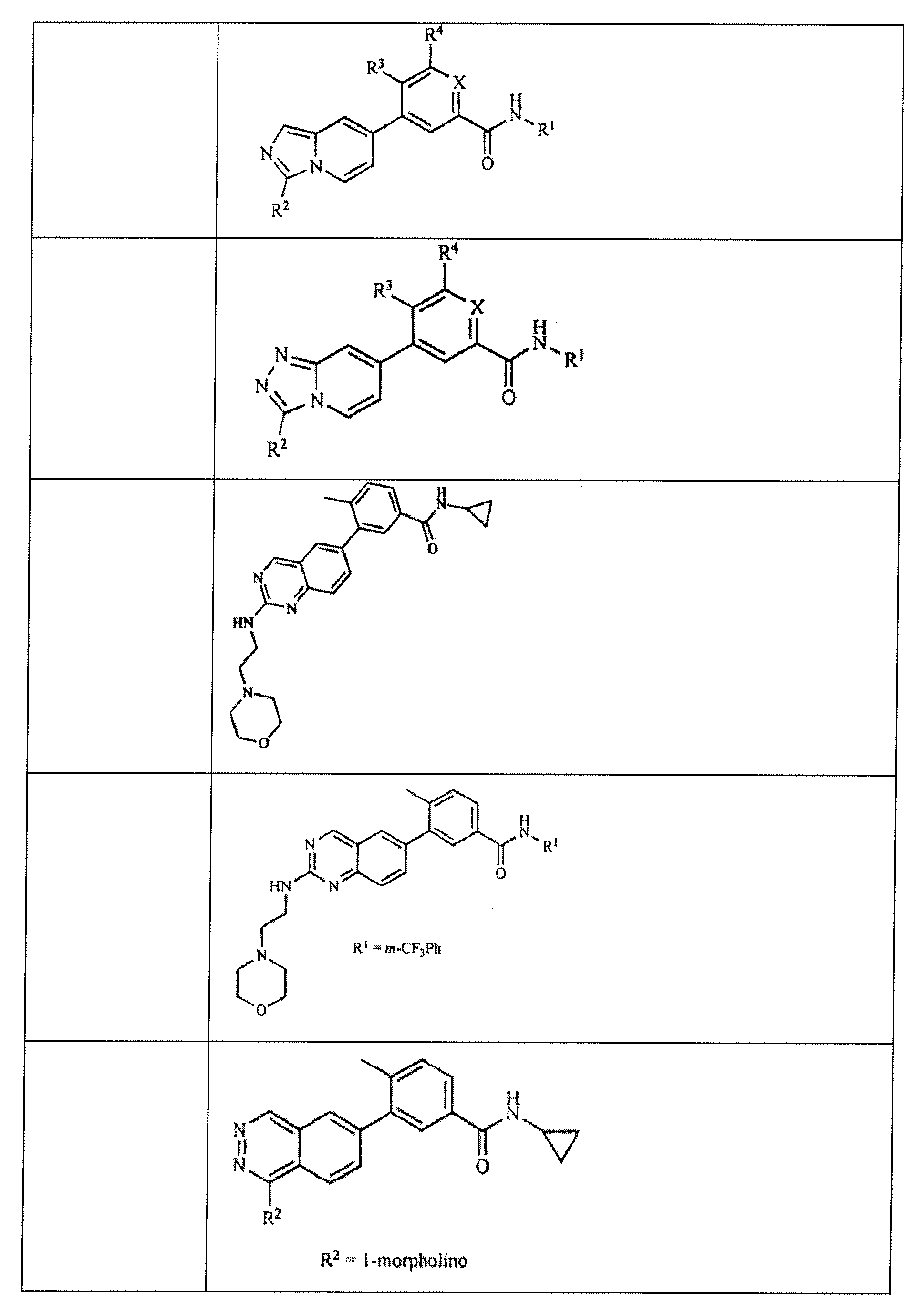

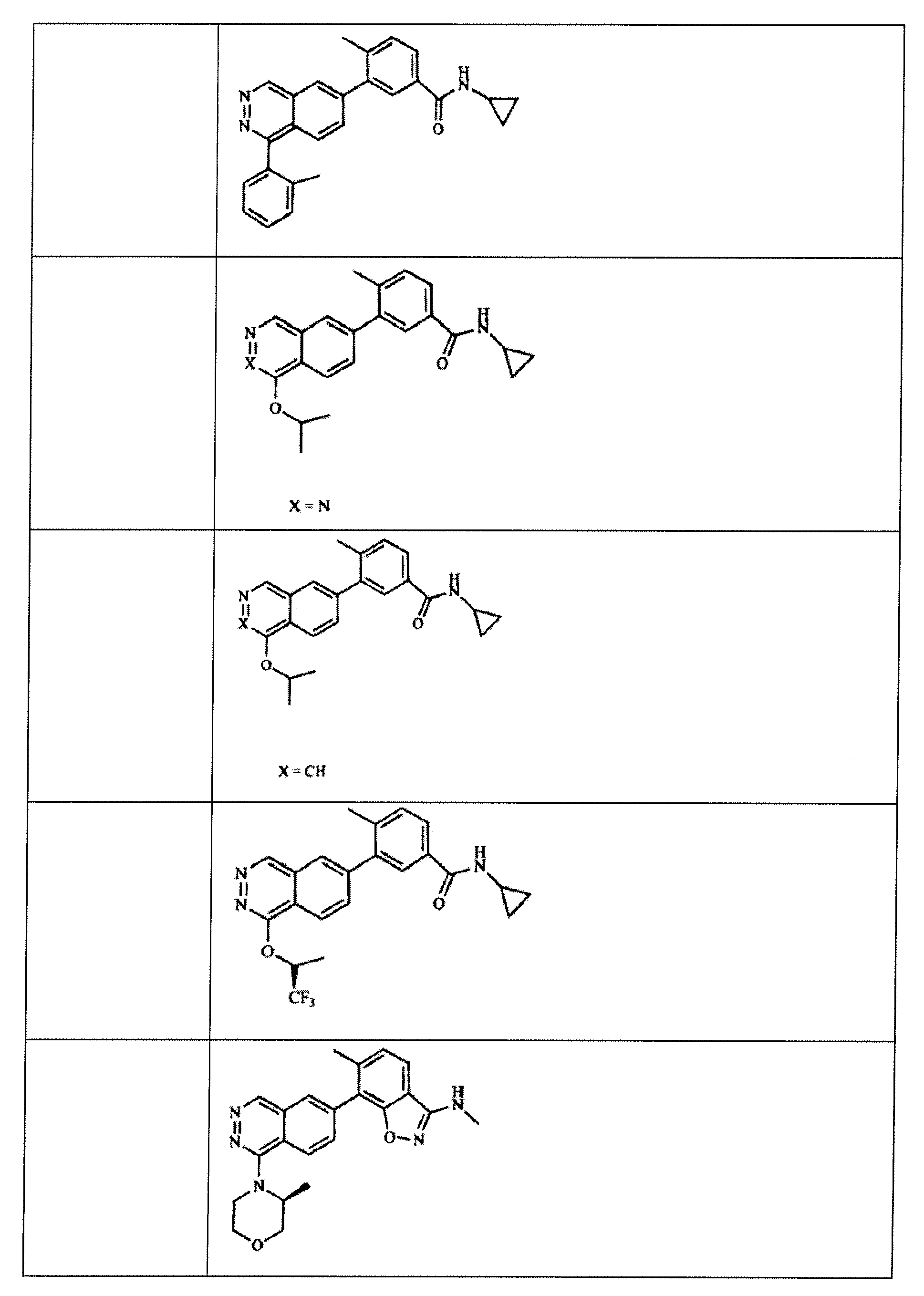

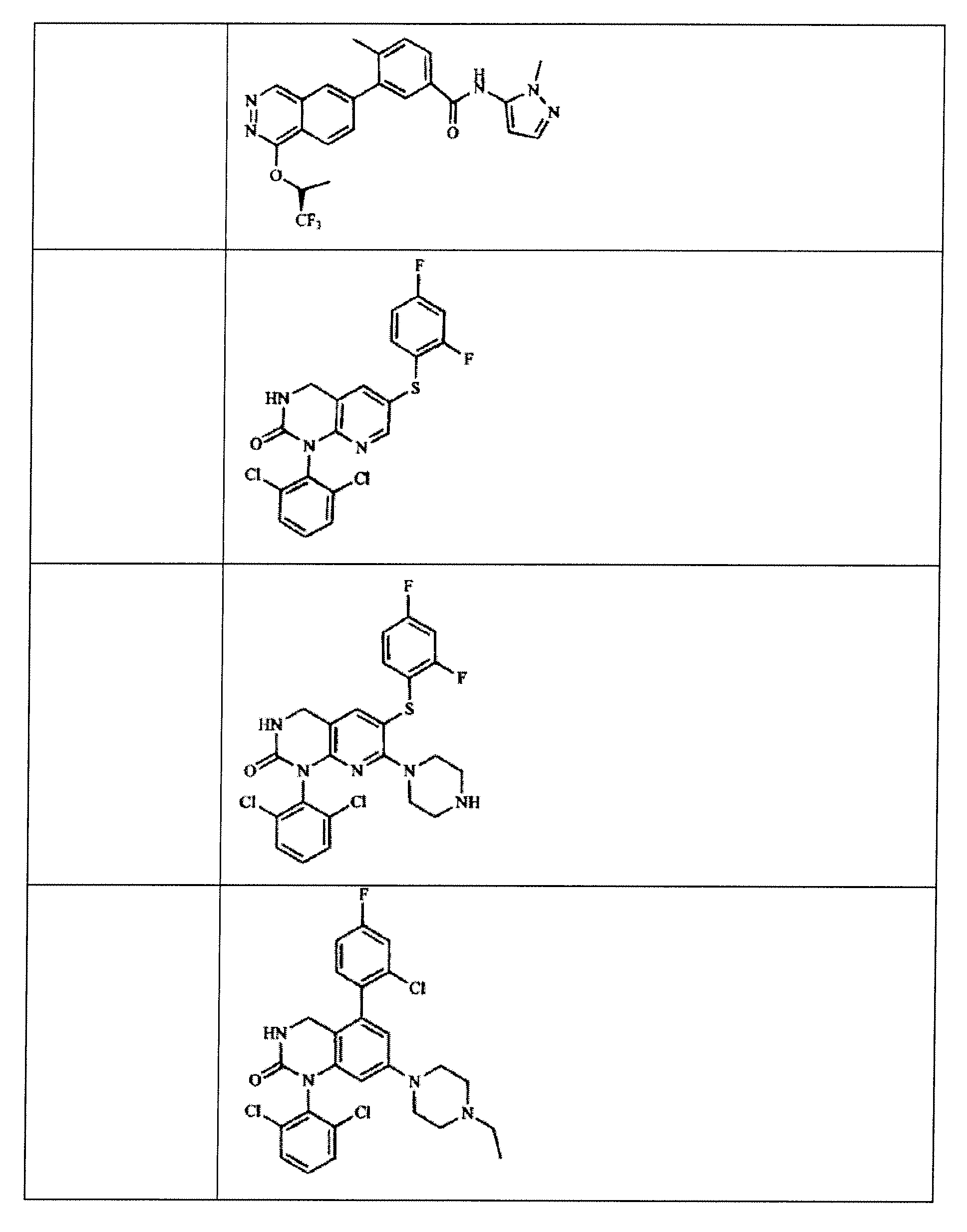

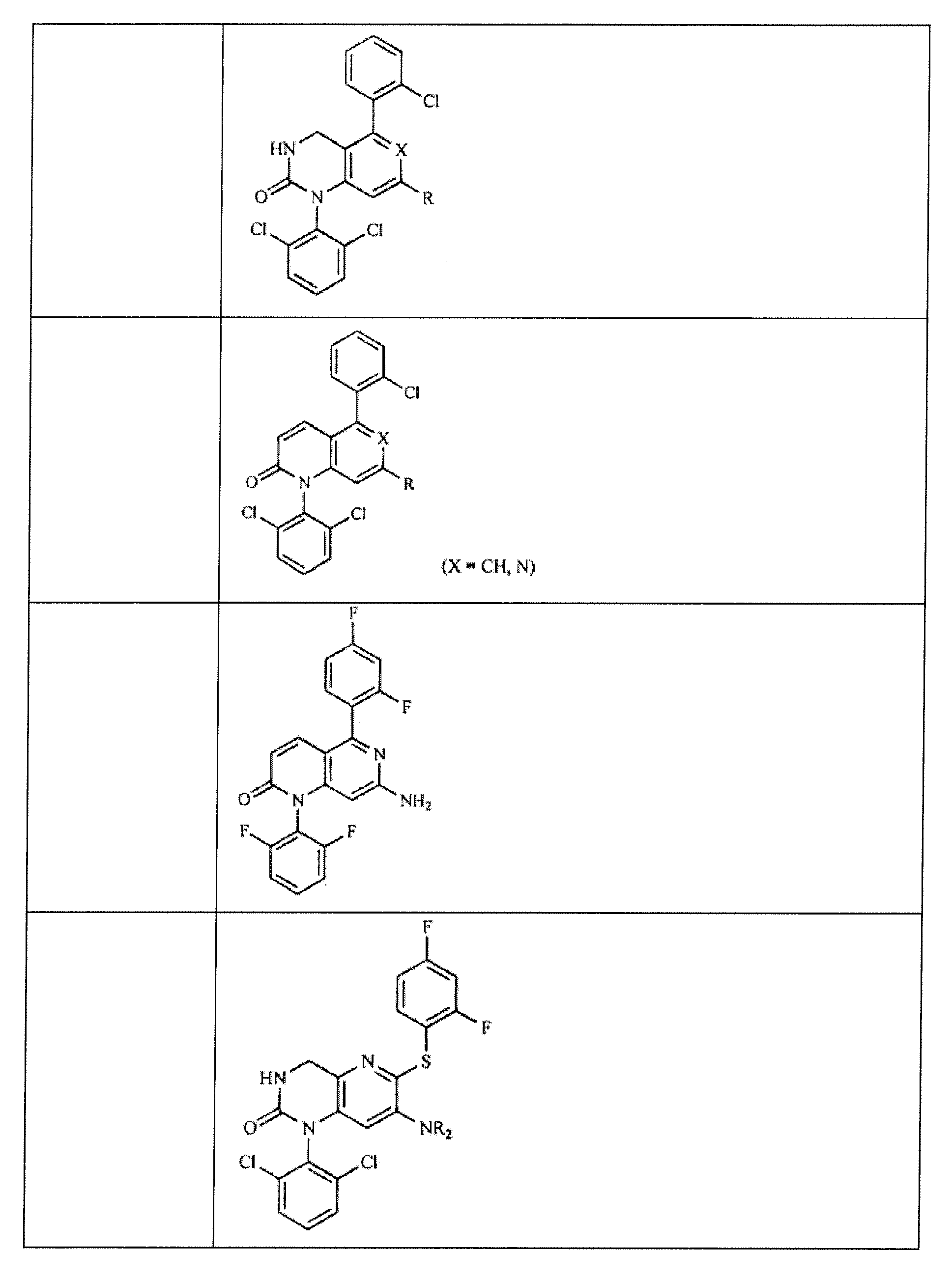

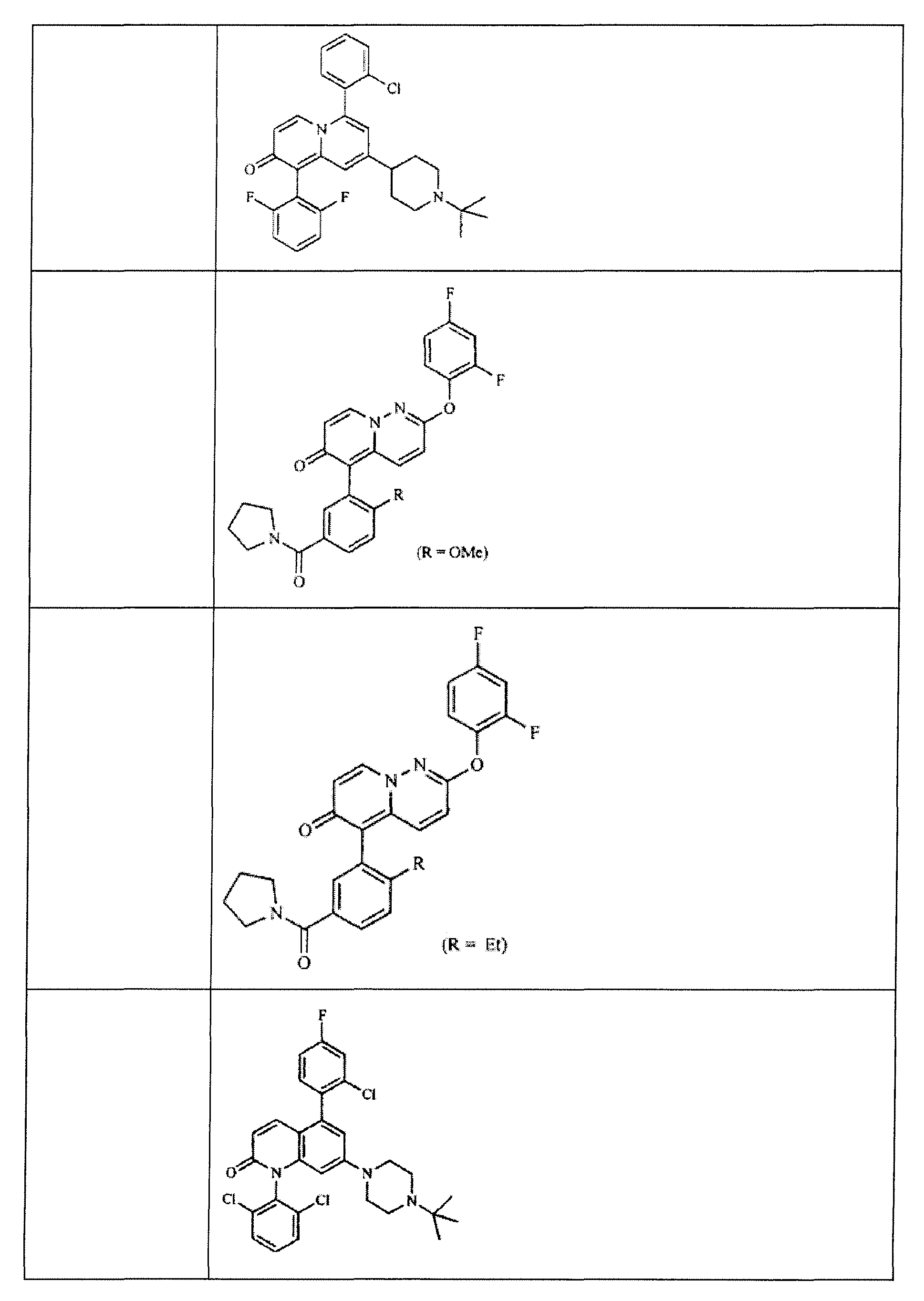

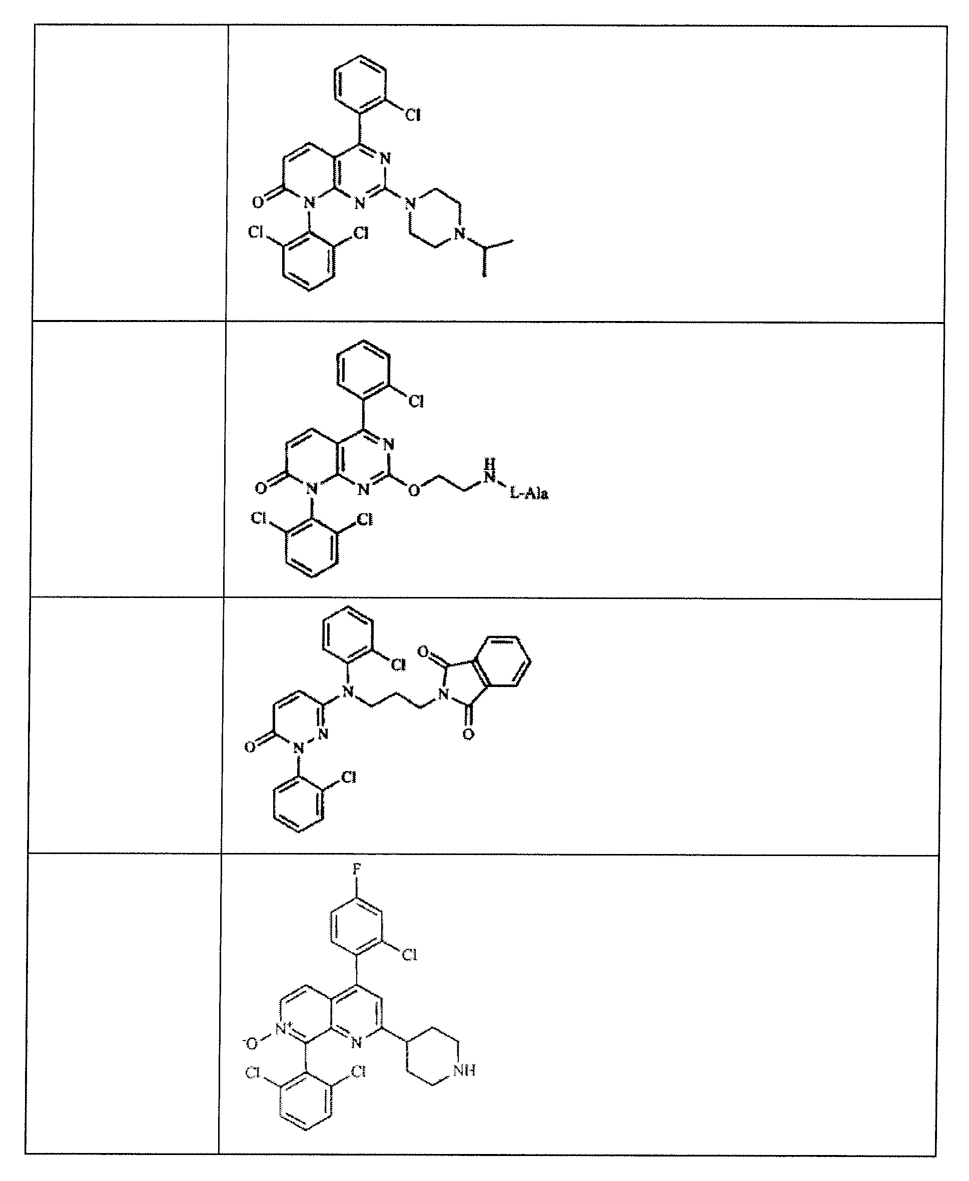

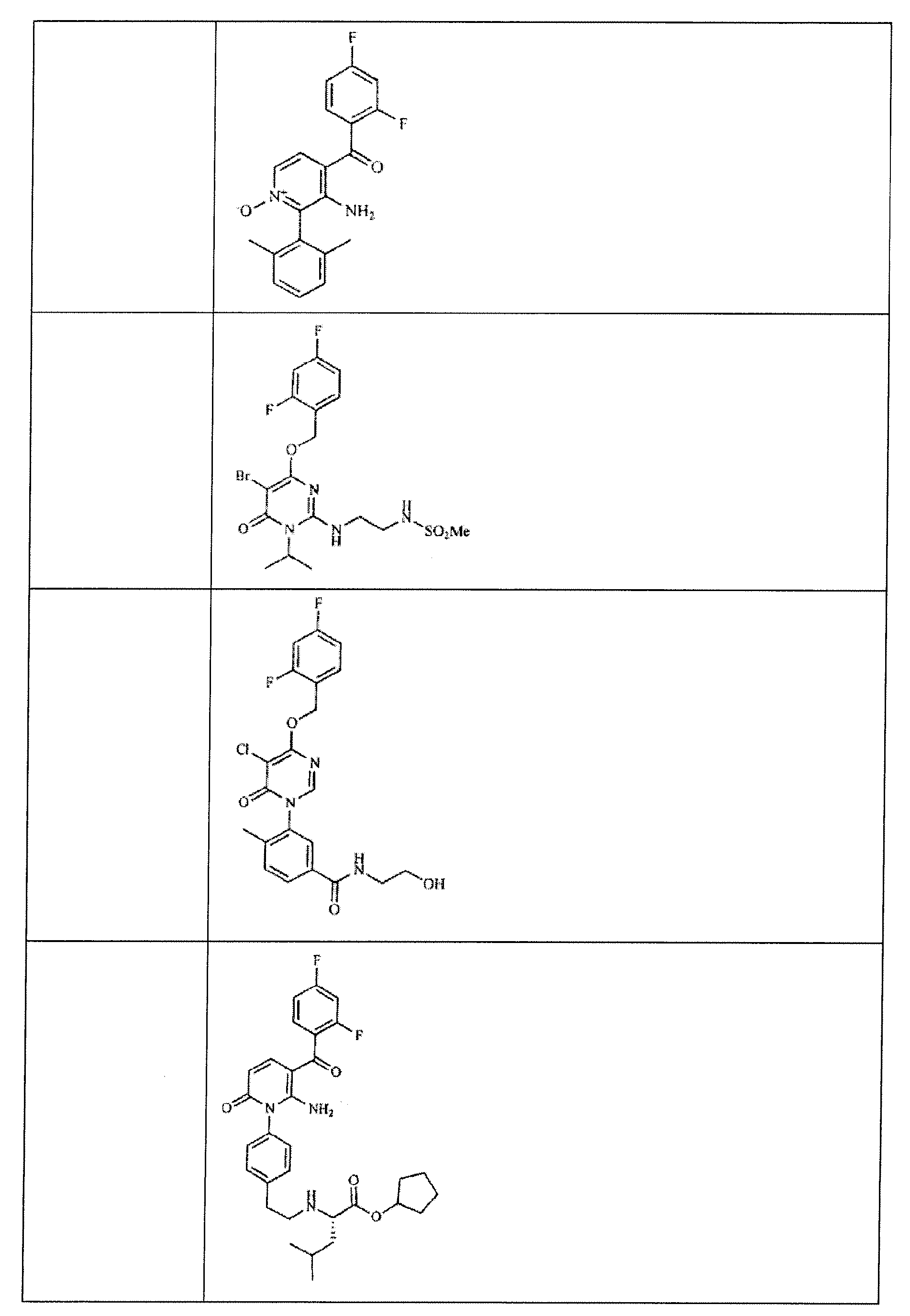

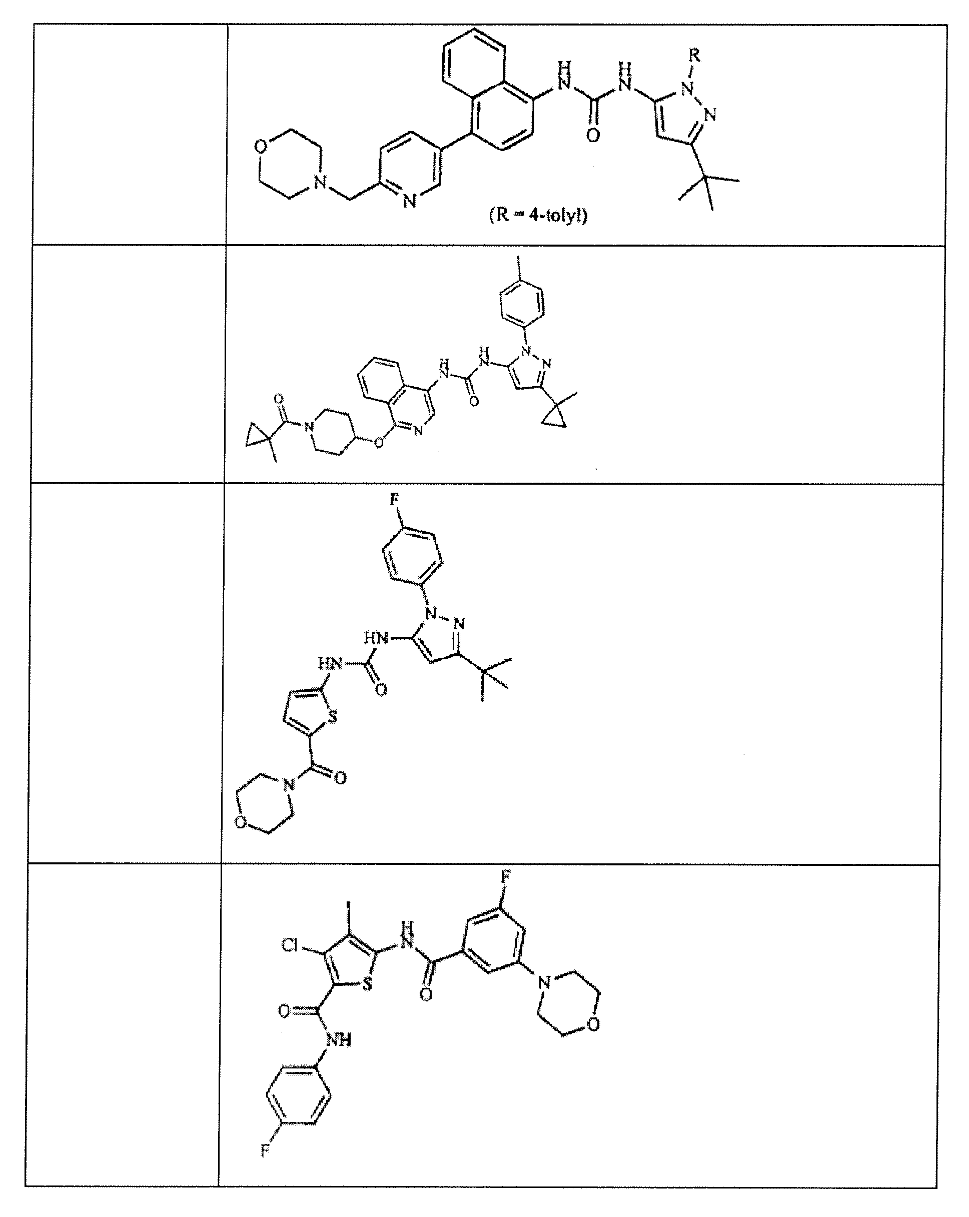

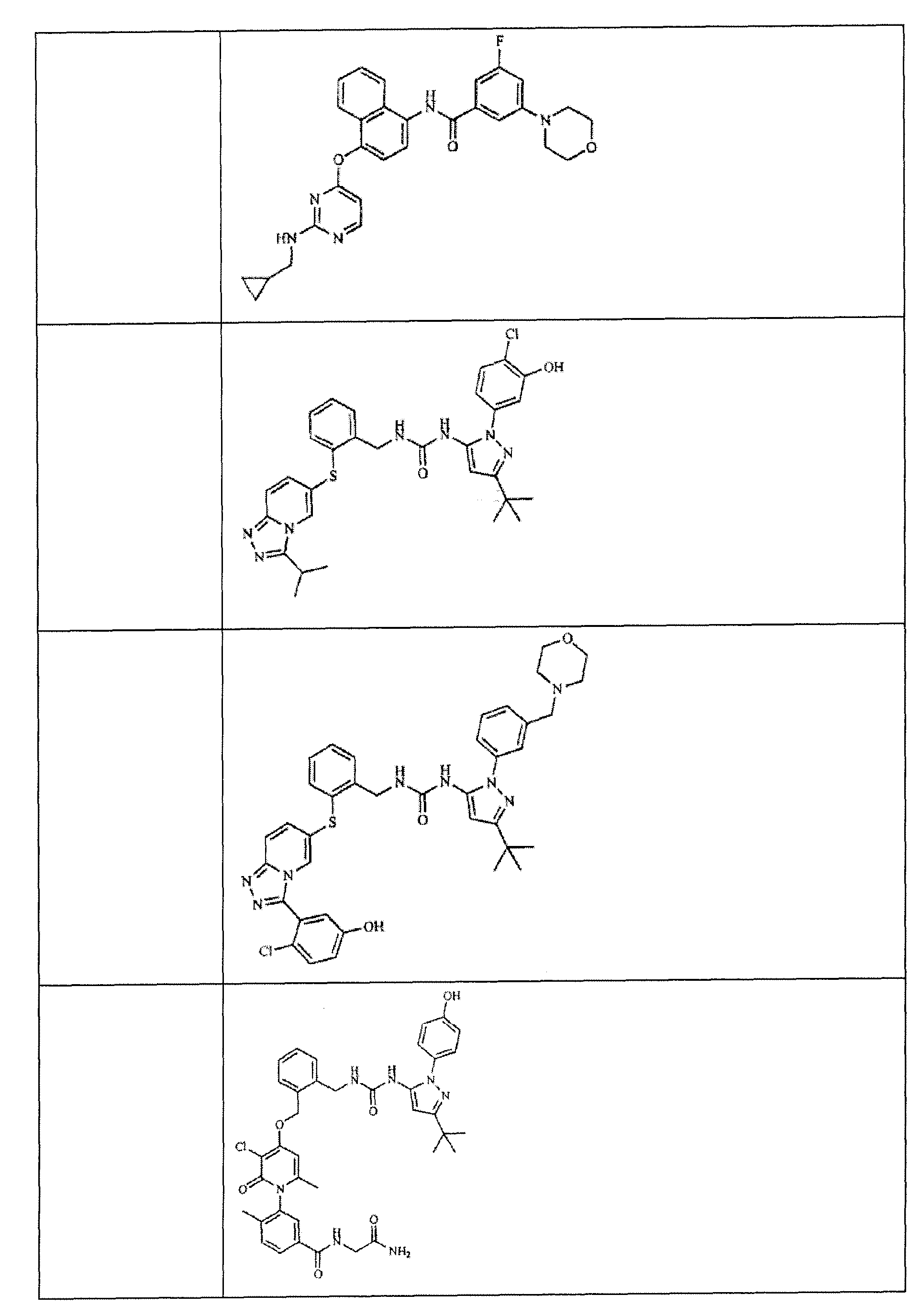

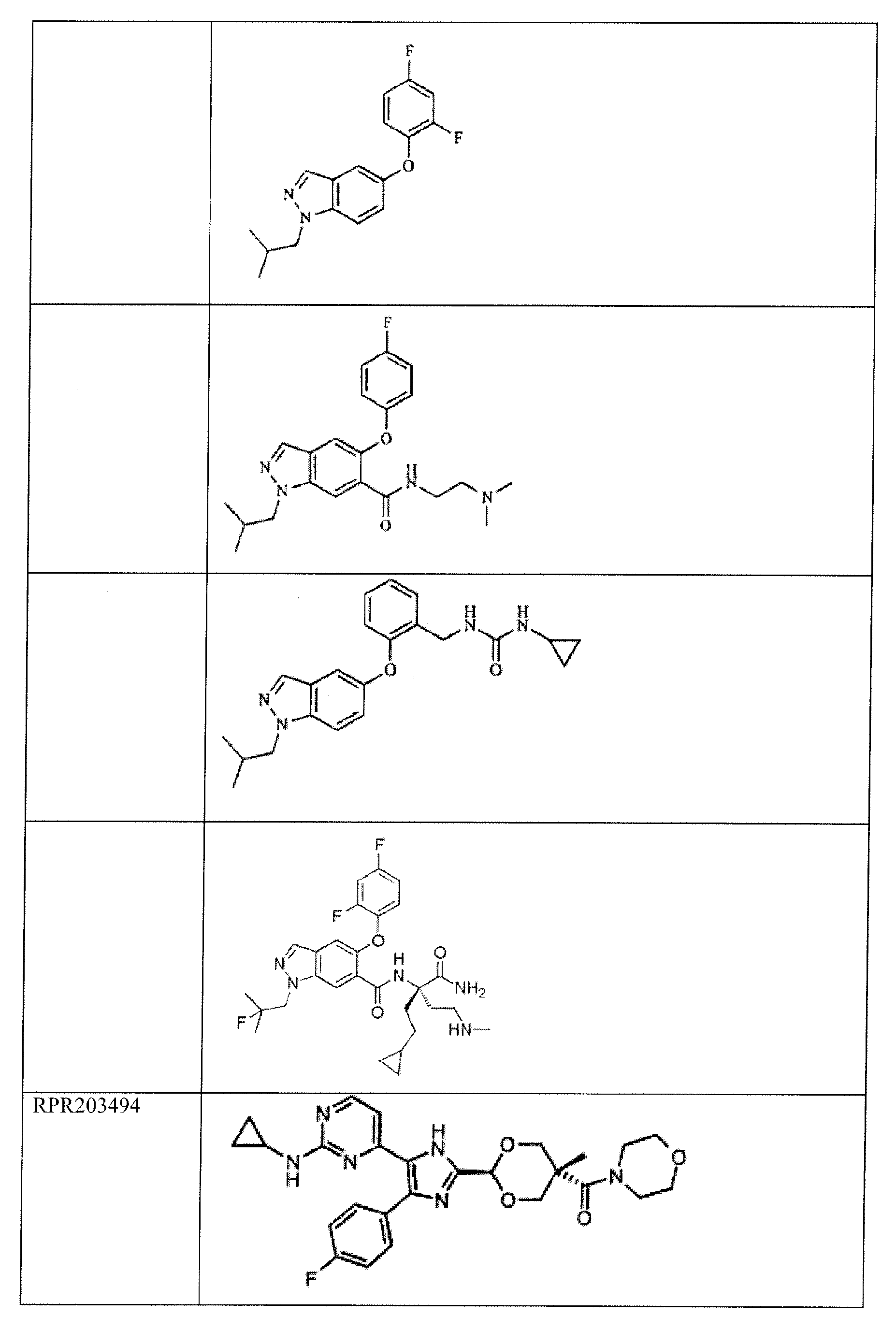

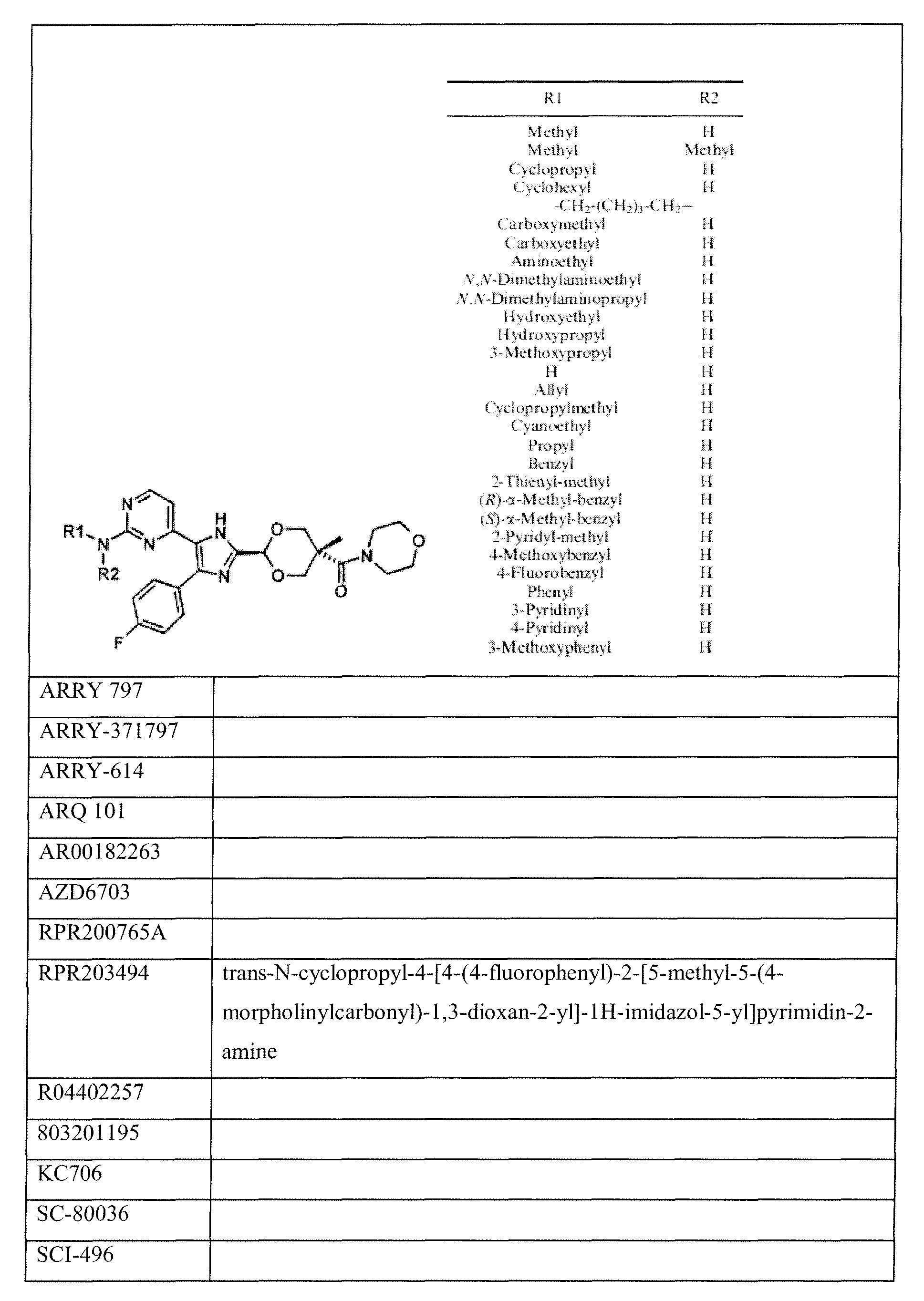

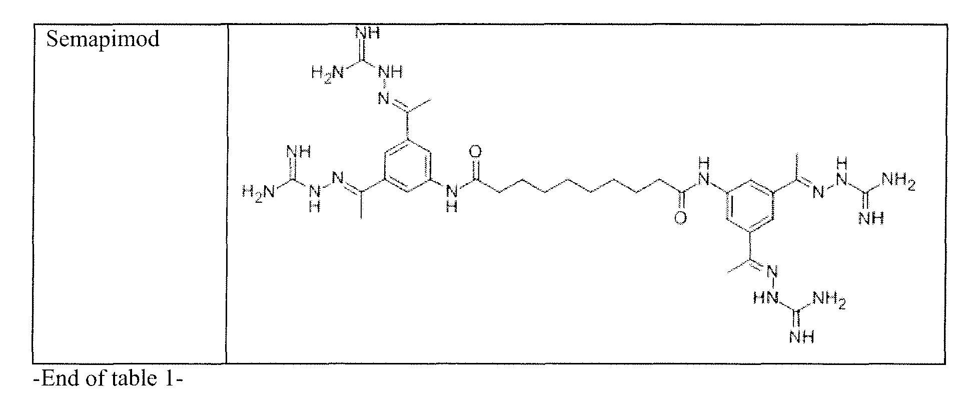

It was found in vitro assays using cultivated liver cancer cells of both murine and human origin, respectively, that the efficacy of Sorafenib against liver cancer cells was increased in the presence of an inhibitor of Mapk14 activity. As examples for inhibitors of Mapk14 activity, BIRB-796, SB 202190, or SX 011 were used, which are kinase inhibitors that act upon and/or are specific for Mapk14 protein.

### Detailed description of the invention

The invention is now described by way of examples with reference to the figures, wherein
- Figure 1 schematically shows the constructs used for genetically manipulating mice for generation of liver carcinomas with concurrent expression of shRNAs which can be non-specific or specific for the mRNA encoding Mapk14,
- Figure 2 shows the survival rates of mice expressing different shRNA molecules with and without administration of Sorafenib,
- Figure 3 shows the detected levels of mRNA specific for Mapk14 with expression of shRNAs which are non-specific or specific for the mRNA encoding Mapk14,
- Figure 4 shows a Western blot with specific detection for Mapk14 protein levels from cells expressing shRNA with or without specificity for Mapk14 mRNA, and with detection for α-tubulin as a loading control,
- Figure 5 shows micrographs and GFP images of explanted mouse livers with and without administration of Sorafenib and expression of shRNAs specific for Mapk14 mRNA and controls,
- Figure 6 schematically depicts the mechanism of the doxycycline dependent transcription of shRNAs and the GFP marker gene.
- Figure 7 shows the survival rates of mice treated with Sorafenib or a control compound (carrier) in the presence of the shRNA specific for the mRNA encoding Mapk14, (which transcription was activated 5 days after tumor development) and in the absence of the shRNA specific for the mRNA encoding Mapk14, as well as control shRNAs.
- Figure 8 shows the number of cells (as an indicator of proliferative activity) in an in vitro assay in which murine liver cancer cells were treated with Sorafenib in combination with the Mapk14 inhibitor BIRB-796,
- Figure 9 shows the number of cells (as an indicator of proliferative activity) in an in vitro assay, in which a human liver cancer cell line was treated with Sorafenib in combination with the inhibitor of Mapk14 BIRB-796,
- Figure 10 shows a picture of cell staining (cell density as an indicator of proliferative activity) (crystal violet assay) of a human liver cancer cell line after treatment of Sorafenib in combination with the Mapk14 - specific inhibitor BIRB-796,
- Figure 11 shows the quantification of the number of dead cells (human liver cancer cell line) after treatment with Sorafenib in combination with the Mapk14 - specific inhibitor BIRB-796 and controls, respectively,
- Figure 12 shows the number of cells in an in vitro assay in which a murine hepatocyte cancer cell line was treated with Sorafenib in combination with the Mapk14 inhibitor SB202190,
- Figure 13 shows the number of cells in an in vitro assay, in which a human liver cancer cell line was treated with Sorafenib in combination with the inhibitor of Mapk14 SB202190,
- Figure 14 shows a picture of cell staining (cell density as a marker for proliferative activity) of a human liver cancer cell line after treatment of Sorafenib in combination with the Mapk14 - specific inhibitor SB202190,
- Figure 15 shows the quantification of the number of dead cells (human liver cancer cell line) after treatment with Sorafenib in combination with the Mapk14 - specific inhibitor SB202190 and controls, respectively,
- Figure 16 shows the number of cells in an in vitro assay in which a murine liver cancer cell line was treated with Sorafenib in combination with the Mapk14 inhibitor SX011, and
- Figure 17 shows the the number of cells in an in vitro assay, in which a human liver cancer cell line was treated with Sorafenib in combination with the inhibitor of Mapk14 SX011.

### Example 1: Treatment of liver cancer in vivo

As a representative of a human patient having liver cancer, a genetically manipulated mouse model (p19^{Arf-/-}*)* was generated, in which liver cancers were induced by a constitutive expression of the oncogenic NrasG12V mutant.

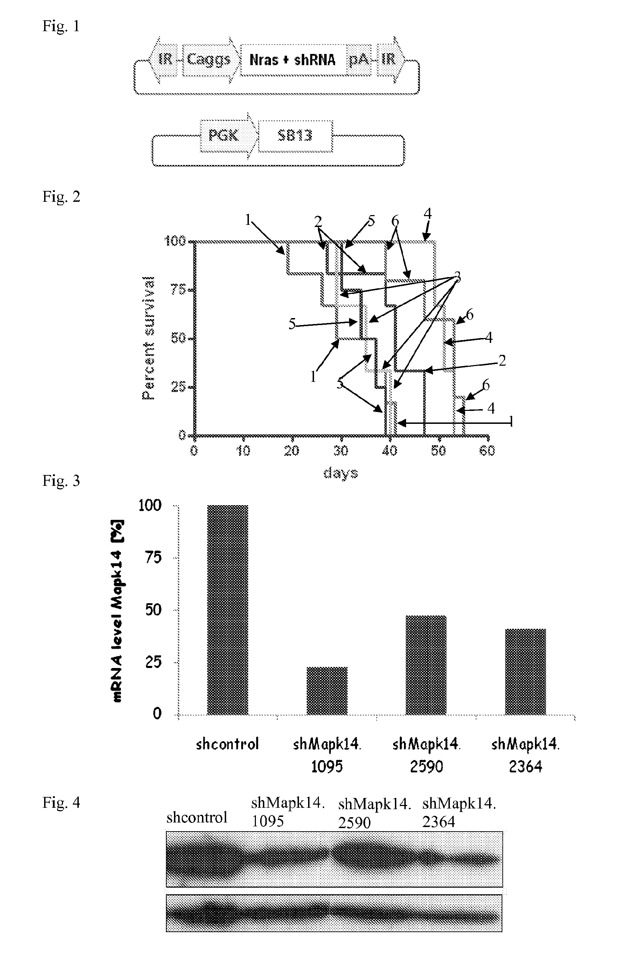

The nucleic acid construct for generating liver carcinomas is schematically shown in Figure 1, containing an expression cassette, wherein the coding sequence for NrasG12V is arranged between a 5' promoter sequence and a 3' polyadenylation site, which expression cassette is flanked by two inverted repeat elements (IR). For intrahepatic delivery of the expression cassette, this nucleic acid construct was administered in combination with a nucleic acid construct containing an expression cassette for the transposase sleeping beauty 13 (SB 13) under the control of the constitutive phosphoglycerate kinase promoter (PGK). The mouse model receiving both nucleic acid constructs shown in Figure 1 was p19^{Arf-/-}, providing the genetic background sufficient for constitutive generation of murine liver cancer. Nucleic acid constructs were introduced into experimental mice using hydrodynamic tail vein injection.

Mice that were genetically conditioned to develop liver cancer were treated with a pharmaceutical composition containing Sorafenib in a carrier, and carrier alone as a control.

The expression cassette in addition to the coding sequence for Nras12V contains the coding sequence for a short hairpin RNA as an example for an siRNA/shRNA. As an shRNA, a non-specific shRNA (shcontrol) was used, an shRNA specific for the murine mRNA encoding murine Mapk14 (shMapk14.1095), and an alternative shRNA specific for the murine mRNA encoding Mapk14 (shMapk14.2590). For each mouse model having one of the nucleic acid constructs, mice were mock-treated with carrier, or with Sorafenib alone.

The survival curves are shown in Figure 2. A liver cancer patient without a Mapk14 inhibitor is represented by the non-specific shRNA (1, shcontrol + carrier) and with the known treatment of Sorafenib (2, shcontrol + Sorafenib) according to the mouse model has an increased survival rate, whereas both mouse models in which the Mapk14 activity is inhibited by the shRNA specific for the mRNA encoding Mapk14 in combination with Sorafenib (4, shMapk14.1095 + Sorafenib, and 6, shMapk14.2590 + Sorafenib) have a significantly increased survival rate. In contrast, the inactivation of Mapk14 alone by an shRNA, i.e. without treatment by administration of Sorafenib (3, shMapk14.1095 + carrier and 5, shMapk14.2590 + carrier) have a survival rate essentially corresponding to the survival rate of mouse models without inactivation of Mapk14 (1, 3, 5).

Figure 3 shows the levels of mRNA encoding Mapk14 in relation (%) to the non-specific control shRNA (shcontrol). It can be seen that the expression of each of shMapk14.1095 (SEQ ID NO: 1370) and shMapk14.2590 (SEQ ID NO: 1371) and of shMapk14.2364, which hybridize to the mRNA encoding Mapk14 results in a reduction of the mRNA encoding Mapk14 in the liver tissue.

Figure 4 shows Western blot analyses of murine liver cancer cells with specific detection of Mapk14 and α-tubulin as a loading control. This analysis shows that the expression level of Mapk14 is significantly reduced by expression of shRNA specific for the mRNA encoding Mapk14, exemplified by shMapk14.1095, shMapk14.2590, and shMapk14.2364, whereas there is a drastically higher level of Mapk14 expression in the presence of shRNA (shcontrol) expression.

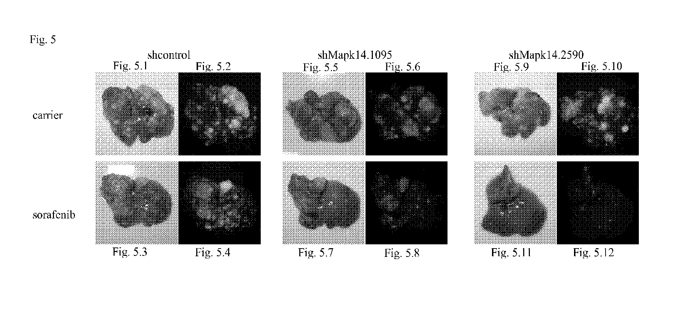

Figure 5 shows photographs of livers explanted from the mouse model and the respective GFP - imaging of explanted mouse livers, confirming visually that in mouse models receivingno Sorafenib (top row, Figs. 5.1, 5.2, 5.5, 5.6, 5.9, and 5.10) (carrier), the expression of a non-specific shRNA (shcontrol, Figs. 5.1, 5.2) or of an shRNA inactivating Mapk14 (shMapk14.1095, shMapk14.2590, Figs. 5.5, 5.6, 5.9, and 5.10) essentially do not reduce the development of liver cancer.

In a further experiment it was observed that in further control mice which did not express an shRNA, a similar tumor development and a similar survival rate was found as for the mice expressing the non-specific shRNA (shcontrol).

The lower row of pictures of Figure 5 (Figs. 5.3, 5.4, 5.7, 5.8, 5.11, and 5.12) shows that the inactivation of Mapk14, in this assay obtained in vivo by expression of an shRNA specific for the mRNA encoding Mapk14 (5.7, 5.8, 5.11, and 5.12) in the presence of treatment with Sorafenib drastically reduces the occurrence of liver cancer both in relation to the treatment with Sorafenib alone (Figs. 5.3 and 5.4) and in relation to expression of an inhibitory shRNA alone (Figs. 5.5, 5.6, 5.9, and 5.10).

The GFP imaging correlates with expression of NrasG12V positive tumors. Decreased GFP activity is observed for shRNAs shMapk14.1095 and shMapk14.2590 in the presence of Sorafenib, indicating reduction of cancer cells that were induced by the expression of Nras.

### Example 2: Treatment of liver cancer

Again, mouse models were used for representing human liver cancer patients in treatment using Sorafenib in combination with an agent inhibiting the activity of Mapk14. As an example for a pharmaceutical agent for the inactivation of the activity of Mapk14, an shRNA (shMapk14.1095) specific for the murine mRNA encoding Mapk14 was used. The shRNA was provided by controlled expression using an expression cassette which is under the control of a tetracycline response element (TRE) of the otherwise constitutive viral promoter (P_{minCMV}). The expression cassette contains the coding sequence for a shRNA molecule and the coding sequence of a GFP marker gene. The nucleic acid construct containing the expression cassette for the shRNA molecule and the coding sequence of a GFP marker gene under the control of the Tet - inducible promoter also contains a constitutive expression cassette for rtTA (rtetRVP16), which in the presence of Doxycycline (DOX) binds to the TRE and activates the promoter of the expression cassette encoding a fusion of shRNA and GFP.

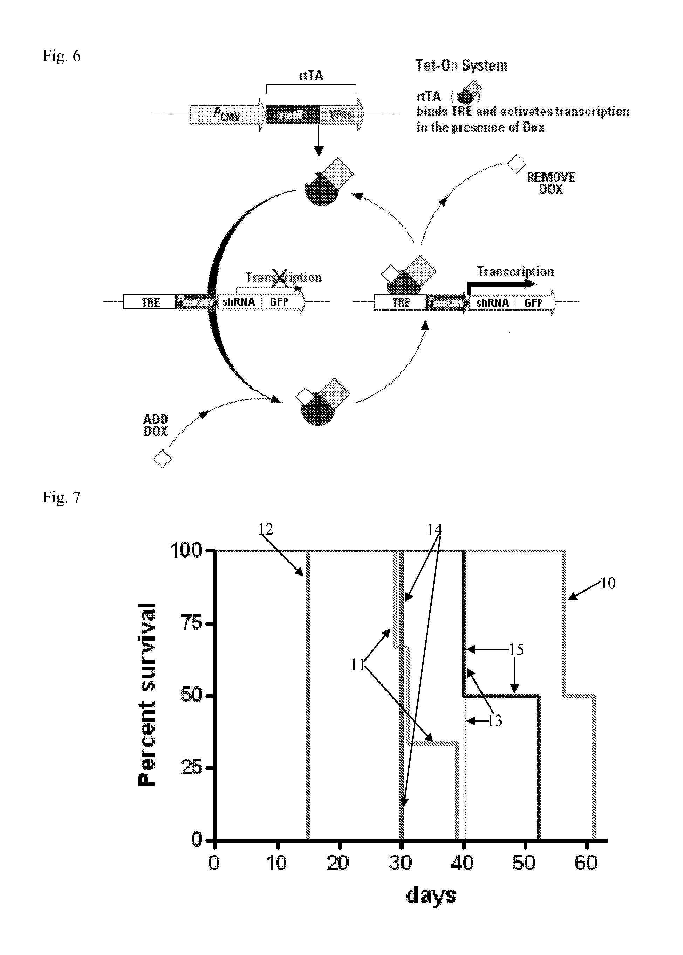

Figure 6 schematically shows the nucleic acid constructs and the mechanism for inducing transcription of shRNA and GFP encoding sequence.

Generally, the nucleic acid construct of Figure 6 is introduced into experimental mice via retroviral infection of cancer cells which after selection are injected into the liver of wildtype mice. The injected mice then develop liver carcinomas in which Mapk14 can be conditionally inactivated by inducing transcription of the shRNA by addition of DOX.

Figure 7 shows the result of an experiment using the expression of an shRNA specific for the mRNA encoding murine Mapk14, namely shMapk14.1095, or a non-specific shRNA (shcontrol). The administration of DOX induces inactivation of Mapk14 by inducing transcription of the shRNA and GFP. The administration of DOX in combination with Sorafenib significantly increased survival rates (10, shMapk14.1095 + DOX + Sorafenib), whereas in the absence of the inactivation of Mapk14, represented by the lack of induction of shMapk14 in the absence of DOX (14, shMapk14.1095 - DOX + Sorafenib) or in the presence of a non-specific shRNA (12, shcontrol + DOX + Sorafenib) showed a lower survival rate as expected for treatment by Sorafenib alone. For control, the absence of a specific inhibitory shRNA and without Sorafenib (15, shMapk14.1095 - DOX - Sorafenib), in the absence of non-specific shRNA and without Sorafenib (13, shcontrol - DOX - Sorafenib), and with presence of Mapk14 specific shRNA without Sorafenib but carrier (11, shMapk14.1095 + DOX + carrier) were tested.

### Example 3: Inactivation of Mapk14 by a specific kinase inhibitor increases the efficacy of Sorafenib against liver cancer cell lines

As an example for a medicament containing Sorafenib in combination with an inhibitor specific for Mapk14 kinase, BIRB-796, SB 202190 and SX 001 were tested in an in vitro assay. These in vitro experiments show the increased efficacy of Sorafenib when administered in combination with an inhibitor of Mapk14 kinase protein on the example of cultivated murine and human liver cancer cell lines.

In the assay, murine liver cancer cells (Nras arf-/-) were cultivated. Cultivated cells were treated with 10 µM Sorafenib, 2 µM BIRB, 12 µM BIRB and with a combination of 10 µM Sorafenib + 2 µM BIRB, or a combination of 10 µM Sorafenib +12 µM BIRB. For each of these assays, a parallel assay was made, replacing Sorafenib or Mapk14 inhibitors by formulation agents without pharmaceutical active agent (carrier).

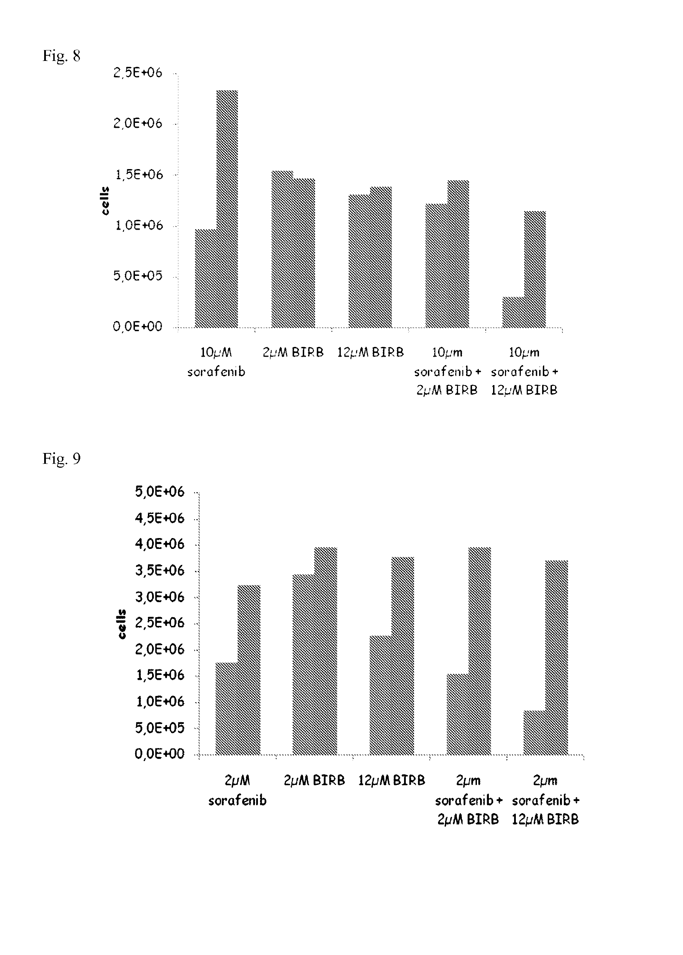

Figure 8 shows the results of the viable cell count after two days of treatment of the murine cancer cells and Figure 9 shows the viable cell count after 7 days of treatment of the cultivated human liver cancer cell line (Hep3B) with the combinations of 2 µM Sorafenib, 2 µM BIRB, 12 µM BIRB, a combination of 2 µM Sorafenib +2 µM BIRB , or a combination of 2 µM Sorafenib +12 µM BIRB, respectively, in the left columns, whereas samples without pharmaceutical active agent (carrier) are shown as right hand columns. These results show that the activity of Sorafenib against liver cancer cells can be reproduced in vitro with cultivated murine and human liver cancer cells, respectively. In detail, the presence of 10 µM or 2 µM Sorafenib as the only pharmaceutical active agent shows a decrease of viable cancer cells after two and seven days, respectively, whereas in murine cells, the inhibitor BIRB-796 only has no significant influence on cultivated murine liver cancer cells, whereas BIRB-796 alone shows a reduction of viable human cancer cells. In murine liver cancer cells, and even more pronounced in human liver cancer cells, the combination of Sorafenib with the inhibitor BIRB-796 shows a marked increase in the efficacy over Sorafenib alone, and the combination of 12µM BIRB-796 with 2µM Sorafenib results in a significant decrease in viable cancer cells compared to the reduction of cancer cells by Sorafenib alone.

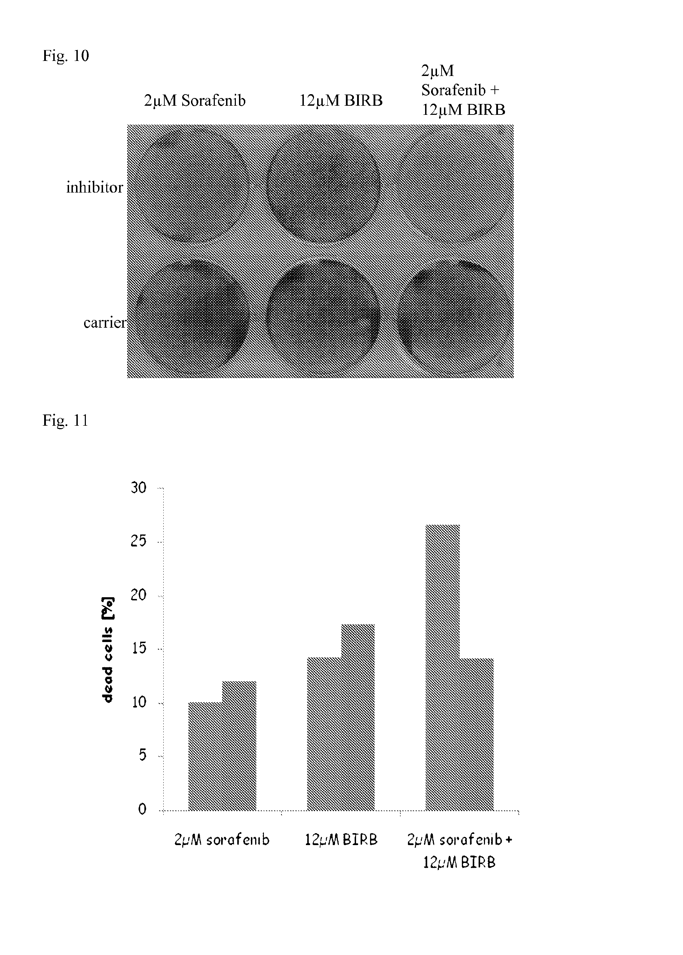

Figure 10 shows crystal violet staining of cultivated human liver cancer cells which are cultivated and treated for 7 days with 2 µM Sorafenib, 12 µM BIRB-796, or a combination of 2 µM Sorafenib +12 µM BIRB-796 (upper row), controls without kinase inhibitor are shown in the lower row (carrier). This view of the culture plates makes it evident that the combination of Sorafenib with an inhibitor for Mapk14, represented by BIRB-796, increases the efficacy of Sorafenib.

The dead cell count of Figure 11, using trypane blue staining of the cultivated human liver cancer cells (Hep3B) shows that the increase in dead cells in the presence of an inhibitor (left hand columns) was more prominent for the combination of Sorafenib with BIRB-796 over Sorafenib or BIRB-796 alone, and over the control (carrier, right hand columns) without a kinase inhibitor.

Similar to the assay for BIRB-796, SB 202190 was tested in an in vitro assay of the murine liver cancer cells (Nras Arf-/-) and the human liver cancer cell line (Hep3B).

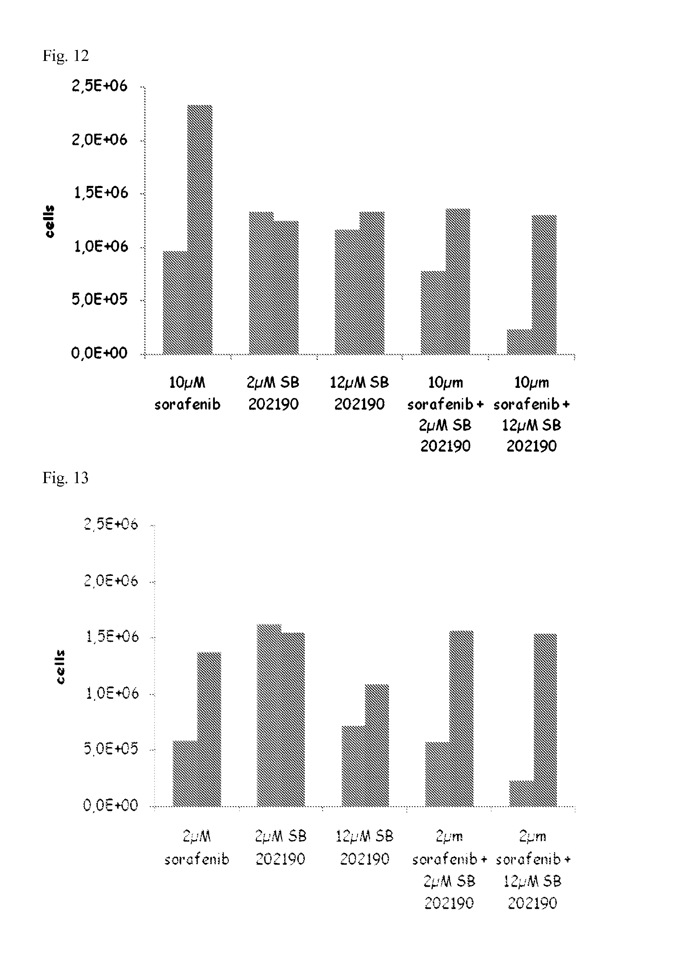

Figure 12 shows the results of the viable cell count after two days of treatment of the murine cancer cells and Figure 13 shows the viable cell count after 7 days of treatment of the cultivated human liver cancer cell line (Hep3B) with the combinations of 2 µM Sorafenib, 2 µM SB202190, 12 µM SB202190, a combination of 2 µM Sorafenib +2 µM SB202190 , or a combination of 2 µM Sorafenib +12 µM SB202190, respectively, in the left columns, whereas samples without pharmaceutical active agent (carrier) are shown as right hand columns.

These results confirm that the activity of Sorafenib against liver cancer cells can be reproduced in vitro with cultivated murine and human cancer cell lines respectively. Further, in murine cancer cells, and even more pronounced in human cancer cells, the combination of Sorafenib with the inhibitor SB202190 shows a marked increase in the efficacy over Sorafenib alone, and the combination of 12µM SB202190 with 2µM Sorafenib results in a significant decrease in viable cancer cells compared to the reduction of cancer cells by Sorafenib alone.

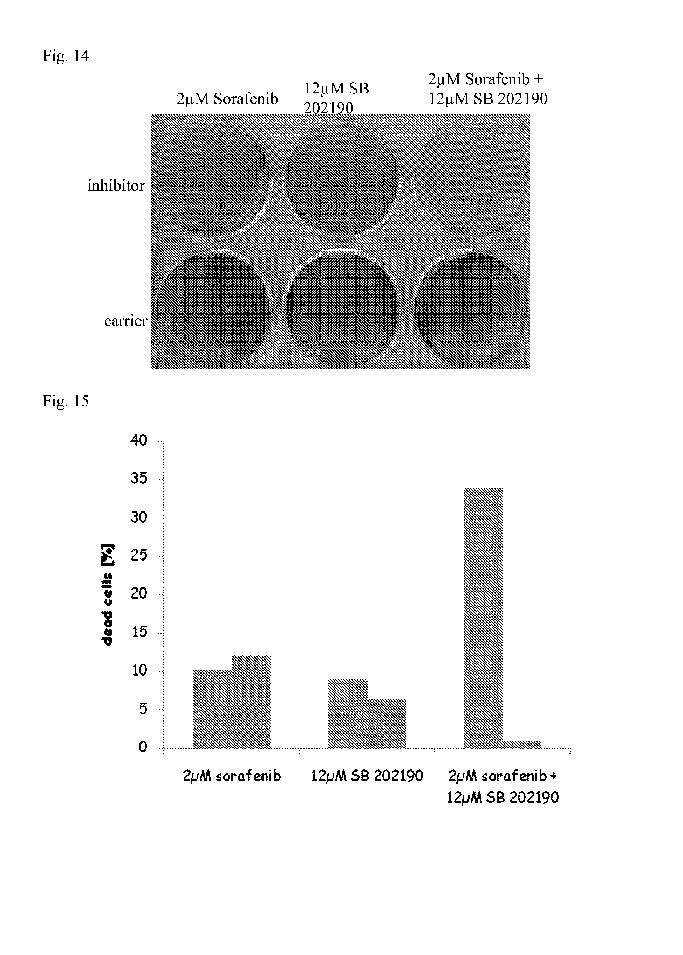

Fig. 14 shows the crystal violet staining of cultivated human liver cancer cells which are cultivated and treated for 7 days with 2 µM Sorafenib, 12 µM SB202190, or a combination of 2 µM Sorafenib +12 µM SB202190 (upper row), controls without kinase inhibitor are shown in the lower row (carrier). This view of the culture plates makes it evident that the combination of Sorafenib with an inhibitor for Mapk14, represented by SB202190, increases the efficacy of Sorafenib.

The dead cell count shown in Fig. 15 (left hand columns indicate counts in presence of inhibitor, right hand columns are control with carrier only) supports the finding that the combination of the Mapk14 inhibitor SB202190 with Sorafenib is significantly more effective than Sorafenib or SB202190 alone.

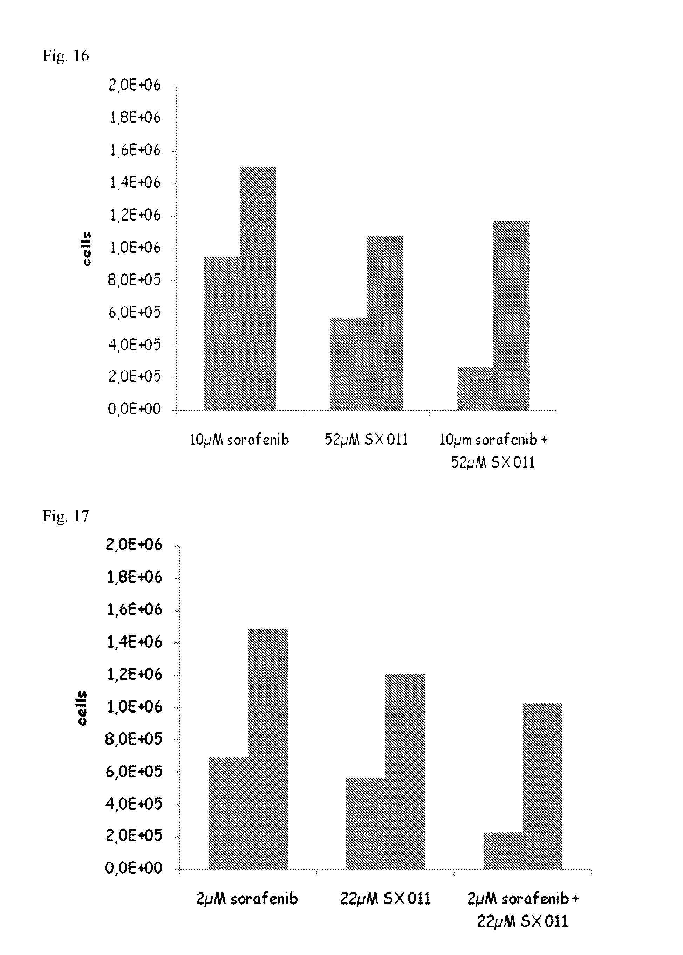

Figure 16 shows the results of the viable cell counts after two days of treatment of the murine cancer cells, and Figure 17 shows the viable cell count after 7 days of treatment of the cultivated human liver cancer cell line (Hep3B) with the combinations of 10 µM or 2 µM Sorafenib, 52 µM or 22 µM SX 011, and a combination of 10 or 2 µM Sorafenib + 52 or 22 µM SX 011 respectively, in the left hand columns, whereas samples without pharmaceutical active agent (carrier) are shown as right hand columns.

These results confirm that the activity of Sorafenib against liver cancer cells can be reproduced in vitro with cultivated murine and human liver cancer cells, respectively. Further, in murine cancer cells, and even more pronounced in human cancer cells, the combination of Sorafenib with the inhibitor SX 011 shows a marked increase in the efficacy over Sorafenib alone, and the combination of SX 011 with Sorafenib results in a significant decrease in viable cancer cells compared to the reduction of cancer cells by Sorafenib alone.

## Claims

1. Pharmaceutical combination comprising Sorafenib, **characterized in that** the composition comprises an inhibitor of the activity of Mapk14 as a medicament for the treatment or prevention of liver cancer.

2. Pharmaceutical combination according to claim 1, **characterized in that** the inhibitor of the activity of Mapk14 is an siRNA containing an oligonucleotide having a nucleotide sequence hybridizing under physiological conditions to the mRNA encoding Mapk14 of SEQ ID NO: 1365, SEQ ID NO: 1366, SEQ ID NO: 1367 or SEQ ID NO: 1368.

3. Pharmaceutical combination according to claim 2, **characterized in that** the oligonucleotide is selected from the group comprising SEQ ID NO: 1 to SEQ ID NO: 1364.

4. Pharmaceutical combination according to one of claims 2 to 3, **characterized in that** the oligonucleotide is comprised as a first section in a nucleic acid construct which contains a second section which is reverse complementary to the first section.

5. Pharmaceutical combination according to one of claims 2 to 4, **characterized in that** the oligonucleotide is arranged under the control of a promoter in an expression cassette.

6. Pharmaceutical combination according to one of claims 2 to 5, characterized that the oligonucleotide is contained in a liposome formulation and/or in a viral vector which is packaged in a viral particle or in a virus-like particle.

7. Pharmaceutical combination according to claim 1, **characterized in that** the inhibitor is selected from the group consisting of the compounds of table 1.

8. Pharmaceutical combination according to one of the preceding claims, **characterized in that** Sorafenib is contained in a formulation for administration separate from a formulation containing the inhibitor of the activity of Mapk14.

9. Pharmaceutical combination according to one of claims 1 to 7, **characterized in that** Sorafenib and the inhibitor of the activity of Mapk14 are contained in one composition.

10. Pharmaceutical composition comprising in a pharmaceutical formulation an inhibitor of the activity of Mapk14 as a medicament for the treatment or prevention of liver cancer, in combination with a separate pharmaceutical formulation comprising Sorafenib as a medicament for the treatment or prevention of liver cancer.

11. Pharmaceutical composition according to claim 10, **characterized in that** the pharmaceutical formulation comprising an inhibitor of the activity of Mapk14 is for administration separate from the administration of the pharmaceutical formulation comprising Sorafenib for the treatment or prevention of liver cancer for administration to a patient.

12. Pharmaceutical composition according to claim 10 or 11, **characterized in that** the inhibitor of the activity of Mapk14 is an siRNA containing an oligonucleotide having a nucleotide sequence hybridizing under physiological conditions to the mRNA encoding Mapk14 of SEQ ID NO: 1365, SEQ ID NO: 1366, SEQ ID NO: 1367 or SEQ ID NO: 1368.

13. Pharmaceutical composition according to one of claims 10 to 12, **characterized in that** the inhibitor is selected from the group consisting of the compounds of table 1.

14. Use of a combination of Sorafenib with an inhibitor of the activity of Mapk14 in a process for producing a medicament for the treatment or prevention of liver cancer.

15. Use according to claim 14, **characterized in that** the inhibitor of the activity of Mapk14 is an siRNA containing an oligonucleotide having a nucleotide sequence hybridizing under physiological conditions to the mRNA encoding Mapk14, the siRNA containing at least one of SEQ ID NO: 1 to SEQ ID NO: 1364, or an inhibitor which is a compound selected from the group consisting of the compounds of table 1.
